# EUROPEAN PATENT SPECIFICATION

(11) **EP 3 452 498 B1**
(45) Date of publication and mention of the grant of the patent: **05.07.2023**
(21) Application number: 17793478.3
(22) Date of filing: 05.05.2017
(51) Int. Cl.: A61K 48/00, C07K 14/47, A61P 21/00, C12N 15/113, C12N 15/86, C12N 9/22, C12N 15/10

(54) **CRISPR/CAS-RELATED COMPOSITIONS FOR TREATING DUCHENNE MUSCULAR DYSTROPHY**
ZUSAMMENSETZUNGEN IN ZUSAMMENHANG MIT CRISPR/CAS ZUR BEHANDLUNG VON DUCHENNE-MUSKELDYSTROPHIE
COMPOSIITONS LIÉES À CRISPR/CAS DESTINÉES À TRAITER LA DYSTROPHIE MUSCULAIRE DE DUCHENNE

(30) Priority: 05.05.2016 US 201662332297 P
(43) Date of publication of application: 13.03.2019
(73) Proprietor: Duke University, Durham, NC 27705 (US); Editas Medicine, Inc., Cambridge, MA 02141 (US)
(72) Inventor: BUMCROT, David, A., Belmont,MA 02478 (US); HUSTON, Nicholas, C., Cambridge,MA 02139 (US); TYCKO, Joshua, C., Cambridge,MA 02139 (US); ROBINSON-HAMM, Jacqueline, Washington, DC 20008 (US); GERSBACH, Charles, A., Durham,NC 27712 (US)
(74) Representative: Harris, Oliver John Richard
(86) International application number: PCT/US2017/031351
(87) International publication number: WO 2017/193029

(56) References cited:
- WO-A1-2014/197740
- WO-A1-2016/161380
- WO-A2-2014/197748
- DAVID G. OUSTEROUT ET AL: "Multiplex CRISPR/Cas9-based genome editing for correction of dystrophin mutations that cause Duchenne muscular dystrophy", NATURE COMMUNICATIONS, vol. 6, no. 1, 18 February 2015 (2015-02-18), XP055575568, DOI: 10.1038/ncomms7244 -& DAVID G OUSTEROUT ET AL: "Multiplex CRISPR/Cas9-based genome editing for correction of dystrophin mutations that cause Duchenne muscular dystrophy - suppl. information", NATURE COMMUNICATIONS, vol. 6, 18 February 2015 (2015-02-18), pages 1-25, XP055627106,
- ARI E. FRIEDLAND ET AL: "Characterization of Staphylococcus aureus Cas9: a smaller Cas9 for all-in-one adeno-associated virus delivery and paired nickase applications", GENOME BIOLOGY, vol. 16, no. 1, 24 November 2015 (2015-11-24), XP055347837, DOI: 10.1186/s13059-015-0817-8
- Ari E Friedland ET AL: "Staphyloccocus aureus Cas9: An Alternative Cas9 for Genome Editing Applications", editas medicine homepage/publications, 11 May 2015 (2015-05-11), XP055455421, DOI: 10.1016/S1525-0016(16)34170-3 Retrieved from the Internet: URL:http://www.editasmedicine.com/data/doc uments/ASGCT%20poster_2015_Ari.pdf [retrieved on 2018-02-28]
- KLEINSTIVER, B ET AL.: 'Broadening the targeting range of Staphylococcus aureus CRISPR-Cas9 by modifying PAM recognition' NATURE BIOTECHNOLOGY 02 November 2015, XP055309933
- DATABASE GENBANK [Online] XP055460248 Database accession no. AF214528.1
- DATABASE GENBANK [Online] XP055460247 Database accession no. X51934.1
- IYOMBE-ENGEMBE, JP ET AL.: 'Efficient Restoration of the Dystrophin Gene Reading Frame and Protein Structure in DMD Myoblasts Using the CinDel Method' MOLECULAR THERAPY--NUCLEIC ACIDS vol. 5, 26 January 2016, page E283, XP055373250

## Description

### SEQUENCE LISTING

The present specification makes reference to a Sequence Listing (submitted electronically as a .txt file named "028193-9231-WO00 As Filed Sequence Listing" on May 5, 2017). The .txt file was generated on May 5, 2017 and is 62,346 bytes in size.

### TECHNICAL FIELD

The present disclosure relates to the field of gene expression alteration, genome engineering and genomic alteration of genes using Clustered Regularly Interspaced Short Palindromic Repeats (CRISPR)/CRISPR-associated (Cas) 9-based systems and viral delivery systems. The present disclosure also relates to the field of genome engineering and genomic alteration of genes in muscle, such as skeletal muscle and cardiac muscle.

### BACKGROUND

Synthetic transcription factors have been engineered to control gene expression for many different medical and scientific applications in mammalian systems, including stimulating tissue regeneration, drug screening, compensating for genetic defects, activating silenced tumor suppressors, controlling stem cell differentiation, performing genetic screens, and creating synthetic gene circuits. These transcription factors can target promoters or enhancers of endogenous genes, or be purposefully designed to recognize sequences orthogonal to mammalian genomes for transgene regulation. The most common strategies for engineering novel transcription factors targeted to user-defined sequences have been based on the programmable DNA-binding domains of zinc finger proteins and transcription-activator like effectors (TALEs). Both of these approaches involve applying the principles of protein-DNA interactions of these domains to engineer new proteins with unique DNA-binding specificity. Although these methods have been widely successful for many applications, the protein engineering necessary for manipulating protein-DNA interactions can be laborious and require specialized expertise.

Additionally, these new proteins are not always effective. The reasons for this are not yet known but may be related to the effects of epigenetic modifications and chromatin state on protein binding to the genomic target site. In addition, there are challenges in ensuring that these new proteins, as well as other components, are delivered to each cell. Existing methods for delivering these new proteins and their multiple components include delivery to cells on separate plasmids or vectors which leads to highly variable expression levels in each cell due to differences in copy number. Additionally, gene activation following transfection is transient due to dilution of plasmid DNA, and temporary gene expression may not be sufficient for inducing therapeutic effects. Furthermore, this approach is not amenable to cell types that are not easily transfected. Thus another limitation of these new proteins is the potency of transcriptional activation.

Site-specific nucleases can be used to introduce site-specific double strand breaks at targeted genomic loci. This DNA cleavage stimulates the natural DNA-repair machinery, leading to one of two possible repair pathways. In the absence of a donor template, the break will be repaired by non-homologous end joining (NHEJ), an error-prone repair pathway that leads to small insertions or deletions of DNA. This method can be used to intentionally disrupt, delete, or alter the reading frame of targeted gene sequences. However, if a donor template is provided along with the nucleases, then the cellular machinery will repair the break by homologous recombination, which is enhanced several orders of magnitude in the presence of DNA cleavage. This method can be used to introduce specific changes in the DNA sequence at target sites. Engineered nucleases have been used for gene editing in a variety of human stem cells and cell lines, and for gene editing in the mouse liver. However, the major hurdle for implementation of these technologies is delivery to particular tissues in vivo in a way that is effective, efficient, and facilitates successful genome modification.

Hereditary genetic diseases have devastating effects on children in the United States. These diseases currently have no cure and can only be managed by attempts to alleviate the symptoms. For decades, the field of gene therapy has promised a cure to these diseases.

However technical hurdles regarding the safe and efficient delivery of therapeutic genes to cells and patients have limited this approach. Duchenne Muscular Dystrophy (DMD) is the most common hereditary monogenic disease and occurs in 1 in 3500 males. DMD is the result of inherited or spontaneous mutations in dystrophin gene. Dystrophin is a key component of a protein complex that is responsible for regulating muscle cell integrity and function. DMD patients typically lose the ability to physically support themselves during childhood, become progressively weaker during the teenage years, and die in their twenties. Current experimental gene therapy strategies for DMD require repeated administration of transient gene delivery vehicles or rely on permanent integration of foreign genetic material into the genomic DNA. Both of these methods have serious safety concerns. Furthermore, these strategies have been limited by an inability to deliver the large and complex *DMD* gene sequence.

### SUMMARY OF THE INVENTION

The present invention provides a vector encoding a first guide RNA (gRNA) molecule, a second gRNA molecule, and at least one Cas9 molecule that recognizes a Protospacer Adjacent Motif (PAM) of either NNGRRT (SEQ ID NO: 24) or NNGRRV (SEQ ID NO: 25), wherein the at least one Cas9 molecule is an *S. aureus* Cas9 molecule; and wherein the first gRNA molecule comprises a targeting domain consisting of a nucleotide sequence as set forth in SEQ ID NO: 1, and the second gRNA molecule comprises a targeting domain consisting of a nucleotide sequence as set forth in SEQ ID NO: 2.

In some embodiments, the vector is configured to form a first and a second double strand break in a first and a second intron flanking exon 51 of the human *DMD* gene, respectively, thereby deleting a segment of the dystrophin gene comprising exon 51. In certain embodiments, the segment has a length of about 800-900, about 1500-2600, about 5200-5500, about 20,000-30,000, about 35,000-45,000, or about 60,000-72,000 base pairs.

In certain embodiments, the at least one Cas9 molecule is a mutant *S. aureus* Cas9 molecule.

In certain embodiments, the vector is a viral vector. In certain embodiments, the vector is an Adeno-associated virus (AAV) vector.

The present invention also provides for a cell comprising an above-described vector. The present invention further provides for a composition comprising an above-described vector.

The present invention further provides the above-described vector for use in a medicament.

The present invention further provides the above-described vector for use in the treatment of Duchenne Muscular Dystrophy.

The present invention further provides a CRISPR/Cas9-based system comprising a first guide RNA (gRNA) molecule, a second gRNA molecule, and at least one Cas9 molecule that recognizes a Protospacer Adjacent Motif (PAM) of either NNGRRT (SEQ ID NO: 24) or

NNGRRV (SEQ ID NO: 25), wherein the at least one Cas9 molecule is an *S. aureus* Cas9 molecule; and wherein the first gRNA molecule comprises a targeting domain consisting of a nucleotide sequence as set forth in SEQ ID NO: 1, and the second gRNA molecule comprises a targeting domain consisting of a nucleotide sequence as set forth in SEQ ID NO: 2.

In some embodiments, the CRISPR/Cas-9-based system is configured to form a first and a second double strand break in a first and a second intron flanking exon 51 of the human *DMD* gene, respectively, thereby deleting a segment of the dystrophin gene comprising exon 51. In certain embodiments, the segment has a length of about 800-900, about 1500-2600, about 5200-5500, about 20,000-30,000, about 35,000-45,000, or about 60,000-72,000 base pairs.

In certain embodiments, the at least one Cas9 molecule is a mutant *S. aureus* Cas9 molecule.

The present invention further provides the CRISPR/Cas9-based system for use in a medicament.

The present invention further provides the CRISPR/Cas9-based system for use in the treatment of Duchenne Muscular Dystrophy.

### BRIEF DESCRIPTION OF THE DRAWINGS

Figure 1 depicts deletion efficiency of presently disclosed vectors in HEK293T cells.
Figure 2 depicts deletion efficiency of presently disclosed vectors in DMD myoblasts.
Figure 3 depicts sequencing results of a presently disclosed vector in DMD myoblasts samples.

### DETAILED DESCRIPTION

The genetic constructs, compositions and methods described herein can be used for genome editing, e.g., correcting or reducing the effects of mutations in *dystrophin* gene involved in genetic diseases, e.g., DMD. The genetic constructs (e.g., vectors) comprise at least one pair of guide RNA molecules that provide the DNA targeting specificity for the *dystrophin* gene, and at least one Cas9 molecule.

The presently disclosed subject matter also provides for genetic constructs, compositions and methods for delivering CRISPR/CRISPR-associated (Cas) 9-based system and gRNAs to target the *dystrophin* gene. The presently disclosed subject matter also provides for delivering of the genetic constructs (e.g., vectors) or compositions comprising thereof to skeletal muscle and cardiac muscle. The vector can be an AAV, including modified AAV vectors. The presently disclosed subject matter provides a means to rewrite the human genome for therapeutic applications and target model species for basic science applications.

Gene editing is highly dependent on cell cycle and complex DNA repair pathways that vary from tissue to tissue. Skeletal muscle is a very complex environment, consisting of large myo fibers with more than 100 nuclei per cell. Gene therapy and biology in general have been limited for decades by in vivo delivery hurdles. These challenges include stability of the carrier in vivo, targeting the right tissue, getting sufficient gene expression and active gene product, and avoiding toxicity that might overcome activity, which is common with gene editing tools. Other delivery vehicles, such as direct injection of plasmid DNA, work to express genes in skeletal muscle and cardiac muscle in other contexts, but do not work well with these site-specific nucleases for achieving detectable levels of genome editing.

While many gene sequences are unstable in AAV vectors and therefore undeliverable, CRISPR/Cas systems are stable in the AAV vectors. When CRISPR/Cas systems are delivered and expressed, they remained active in the skeletal muscle tissue. The protein stability and activity of the CRISPR/Cas systems are highly tissue type- and cell type- dependent. These active and stable CRISPR/Cas systems are able to modify gene sequences in the complex environment of skeletal muscle. The presently disclosed subject matter describes a way to deliver active forms of this class of therapeutics to skeletal muscle or cardiac muscle that is effective, efficient and facilitates successful genome modification.

Section headings as used in this section and the entire disclosure herein are merely for organizational purposes and are not intended to be limiting.

### 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the presently disclosed subject matter. The materials, methods, and examples disclosed herein are illustrative only and not intended to be limiting.

Unless otherwise defined herein, scientific and technical terms used in connection with the present disclosure shall have the meanings that are commonly understood by those of ordinary skill in the art. For example, any nomenclatures used in connection with, and techniques of, cell and tissue culture, molecular biology, immunology, microbiology, genetics and protein and nucleic acid chemistry and hybridization described herein are those that are well known and commonly used in the art. The meaning and scope of the terms should be clear; in the event however of any latent ambiguity, definitions provided herein take precedent over any dictionary or extrinsic definition. Further, unless otherwise required by context, singular terms shall include pluralities and plural terms shall include the singular.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "an" and "the" include plural references unless the context clearly dictates otherwise. The present disclosure also contemplates other embodiments "comprising," "consisting of", and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1 , 6.2, 6.3, 6.4, 6.5, 6.6, 6.7, 6.8, 6.9, and 7.0 are explicitly contemplated.

As used herein, the term "about" or "approximately" means within an acceptable error range for the particular value as determined by one of ordinary skill in the art, which will depend in part on how the value is measured or determined, *i.e*., the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

"Frameshift" or "frameshift mutation" as used interchangeably herein refers to a type of gene mutation wherein the addition or deletion of one or more nucleotides causes a shift in the reading frame of the codons in the mRNA. The shift in reading frame may lead to the alteration in the amino acid sequence at protein translation, such as a missense mutation or a premature stop codon.

"Fusion protein" as used herein refers to a chimeric protein created through the joining of two or more genes that originally coded for separate proteins. The translation of the fusion gene results in a single polypeptide with functional properties derived from each of the original proteins.

"Genetic construct" as used herein refers to the DNA or RNA molecules that comprise a nucleotide sequence that encodes a protein. The coding sequence includes initiation and termination signals operably linked to regulatory elements including a promoter and polyadenylation signal capable of directing expression in the cells of the individual to whom the nucleic acid molecule is administered.

As used herein, the term "expressible form" refers to gene constructs that contain the necessary regulatory elements operable linked to a coding sequence that encodes a protein such that when present in the cell of the individual, the coding sequence will be expressed.

"Mutant gene" or "mutated gene" as used interchangeably herein refers to a gene that has undergone a detectable mutation. A mutant gene has undergone a change, such as the loss, gain, or exchange of genetic material, which affects the normal transmission and expression of the gene. A "disrupted gene" as used herein refers to a mutant gene that has a mutation that causes a premature stop codon. The disrupted gene product is truncated relative to a full-length undisrupted gene product.

"Normal gene" as used herein refers to a gene that has not undergone a change, such as a loss, gain, or exchange of genetic material. The normal gene undergoes normal gene transmission and gene expression.

"Nucleic acid" or "oligonucleotide" or "polynucleotide" as used herein means at least two nucleotides covalently linked together. T he depiction of a single strand also defines the sequence of the complementary strand. Thus, a nucleic acid also encompasses the complementary strand of a depicted single strand. Many variants of a nucleic acid may be used for the same purpose as a given nucleic acid. Thus, a nucleic acid also encompasses substantially identical nucleic acids and complements thereof. A single strand provides a probe that may hybridize to a target sequence under stringent hybridization conditions. Thus, a nucleic acid also encompasses a probe that hybridizes under stringent hybridization conditions.

Nucleic acids can be single stranded or double stranded, or may contain portions of both double stranded and single stranded sequence. The nucleic acid can be DNA, both genomic and cDNA, RNA, or a hybrid, where the nucleic acid may contain combinations of deoxyribo- and ribo-nucleotides, and combinations of bases including uracil, adenine, thymine, cytosine, guanine, inosine, xanthine hypoxanthine, isocytosine and isoguanine. Nucleic acids can be obtained by chemical synthesis methods or by recombinant methods.

"Operably linked" as used herein means that expression of a gene is under the control of a promoter with which it is spatially connected. A promoter can be positioned 5' (upstream) or 3' (downstream) of a gene under its control. The distance between the promoter and a gene can be approximately the same as the distance between that promoter and the gene it controls in the gene from which the promoter is derived. As is known in the art, variation in this distance can be accommodated without loss of promoter function.

"Premature stop codon" or "out-of-frame stop codon" as used interchangeably herein refers to nonsense mutation in a sequence of DNA, which results in a stop codon at location not normally found in the wild-type gene. A premature stop codon may cause a protein to be truncated or shorter compared to the full-length version of the protein.

"Promoter" as used herein means a synthetic or naturally-derived molecule which is capable of conferring, activating or enhancing expression of a nucleic acid in a cell. A promoter can comprise one or more specific transcriptional regulatory sequences to further enhance expression and/or to alter the spatial expression and/or temporal expression of same. A promoter can also comprise distal enhancer or repressor elements, which may be located as much as several thousand base pairs from the start site of transcription. A promoter can be derived from sources including viral, bacterial, fungal, plants, insects, and animals. A promoter can regulate the expression of a gene component constitutively, or differentially with respect to cell, the tissue or organ in which expression occurs or, with respect to the developmental stage at which expression occurs, or in response to external stimuli such as physiological stresses, pathogens, metal ions, or inducing agents. Representative examples of promoters include the bacteriophage T7 promoter, bacteriophage T3 promoter, SP6 promoter, lac operator-promoter, tac promoter, SV40 late promoter, SV40 early promoter, RSV-LTR promoter, CMV IE promoter, SV40 early promoter or SV40 late promoter and the CMV IE promoter.

"Skeletal muscle" as used herein refers to a type of striated muscle, which is under the control of the somatic nervous system and attached to bones by bundles of collagen fibers known as tendons. Skeletal muscle is made up of individual components known as myocytes, or "muscle cells", sometimes colloquially called "muscle fibers." Myocytes are formed from the fusion of developmental myoblasts (a type of embryonic progenitor cell that gives rise to a muscle cell) in a process known as myogenesis. These long, cylindrical, multinucleated cells are also called myo fibers. In certain embodiments, "skeletal muscle condition" refers to a condition related to the skeletal muscle, such as muscular dystrophies, aging, muscle degeneration, wound healing, and muscle weakness or atrophy.

"Cardiac muscle" or "heart muscle" as used interchangeably herein means a type of involuntary striated muscle found in the walls and histological foundation of the heart, the myocardium. Cardiac muscle is made of cardiomyocytes or myocardiocytes. Myocardiocytes show striations similar to those on skeletal muscle cells but contain only one, unique nucleus, unlike the multinucleated skeletal cells. In certain embodiments,"cardiac muscle condition" refers to a condition related to the cardiac muscle, such as cardiomyopathy, heart failure, arrhythmia, and inflammatory heart disease.

"Subject" and "patient" as used herein interchangeably refers to any vertebrate, including, but not limited to, a mammal {e.g., cow, pig, camel, llama, horse, goat, rabbit, sheep, hamsters, guinea pig, cat, dog, rat, and mouse, a non-human primate (for example, a monkey, such as a cynomolgous or rhesus monkey, chimpanzee, etc.) and a human). In certain embodiments, the subject is a human. The subject or patient can be undergoing other forms of treatment.

"Target gene" as used herein refers to any nucleotide sequence encoding a known or putative gene product. The target gene may be a mutated gene involved in a genetic disease. In certain embodiments, the target gene is a human *dystrophin* gene. In certain embodiments, the target gene is a mutant humnan *dystrophin* gene.

"Target region" as used herein refers to the region of the target gene to which the gRNA molecule is designed to bind and cleave.

"Variant" used herein with respect to a nucleic acid means (i) a portion or fragment of a referenced nucleotide sequence; (ii) the complement of a referenced nucleotide sequence or portion thereof; (iii) a nucleic acid that is substantially identical to a referenced nucleic acid or the complement thereof; or (iv) a nucleic acid that hybridizes under stringent conditions to the referenced nucleic acid, complement thereof, or a sequences substantially identical thereto. "Variant" with respect to a peptide or polypeptide that differs in amino acid sequence by the insertion, deletion, or conservative substitution of amino acids, but retain at least one biological activity. Variant can also mean a protein with an amino acid sequence that is substantially identical to a referenced protein with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes may be identified, in part, by considering the hydropathic index of amino acids, as understood in the art. Kyte et al., J. Mol. Biol. 157: 105-132 (1982). The hydropathic index of an amino acid is based on a consideration of its hydrophobicity and charge. It is known in the art that amino acids of similar hydropathic indexes may be substituted and still retain protein function. In one aspect, amino acids having hydropathic indexes of ±2 are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide. Substitutions can be performed with amino acids having hydrophilicity values within ±2 of each other. Both the hydrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties.

"Vector" as used herein means a nucleic acid sequence containing an origin of replication. A vector can be a viral vector, bacteriophage, bacterial artificial chromosome or yeast artificial chromosome. A vector can be a DNA or R A vector. A vector can be a self-replicating extrachromosomal vector, e.g., a DNA plasmid. For example, the vector can encode one Cas9 molecule and a pair of gRNA molecules.

### 2. Genetic Constructs for Genome Editing of Dystrophin Gene

The presently disclosed subject matter provides for genetic constructs for genome editing or genomic alteration of a *dystrophin* gene (e.g., human *dystrophin* gene).

In certain embodiments, dystrophin refers to a rod-shaped cytoplasmic protein which is a part of a protein complex that connects the cytoskeleton of a muscle fiber to the surrounding extracellular matrix through the cell membrane. Dystrophin provides structural stability to the dystroglycan complex of the cell membrane that is responsible for regulating muscle cell integrity and function. In certain embodiments, a *dystrophin* gene (or a "*DMD* gene") is 2.2 megabases at locus Xp21. The primary transcription measures about 2,400 kb with the mature mRNA being about 14 kb. 79 exons code for the protein which is over 3500 amino acids.

A presently disclosed genetic construct encodes a CRISPR/Cas9 system that comprises at least one Cas9 molecule and two gRNA molecules. The presently disclosed subject matter also provides for compositions comprising such genetic constructs. The genetic construct can be present in a cell as a functioning extrachromosomal molecule. The genetic construct can be a linear minichromosome including centromere, telomeres or plasmids or cosmids.

The genetic construct can be part of a genome of a recombinant viral vector, including recombinant lentivirus, recombinant adenovirus, and recombinant adenovirus associated virus. The genetic construct can be part of the genetic material in attenuated live microorganisms or recombinant microbial vectors which live in cells. The genetic constructs can comprise regulatory elements for gene expression of the coding sequences of the nucleic acid. The regulatory elements may be a promoter, an enhancer, an initiation codon, a stop codon, or a polyadenylation signal.

The genetic construct is a vector. The vector can be an Adeno-associated virus (AAV) vector, which encodes at least one Cas9 molecule and a pair of two gRNA molecules; the vector is capable of expressing the at least one Cas9 molecule and the at least gRNA molecule, in the cell of a mammal. The vector can be a plasmid. The vectors can be used for *in vivo* gene therapy.

In certain embodiments, an AAV vector is a small virus belonging to the genus Dependovirus of the Parvoviridae family that infects humans and some other primate species.

Coding sequences can be optimized for stability and high levels of expression. In certain instances, codons are selected to reduce secondary structure formation of the RNA such as that formed due to intramolecular bonding.

The vector can further comprise an initiation codon, which can be upstream of the CRISPR/Cas9-based system, and a stop codon, which can be downstream of the CRISPR/Cas9-based system or the site-specific nuclease coding sequence. The initiation and termination codon can be in frame with the CRISPR/Cas9-based system or the site-specific nuclease coding sequence. The vector can also comprise a promoter that is operably linked to the CRISPR/Cas9-based system. The promoter operably linked to the CRISPR/Cas9-based system can be a promoter from simian virus 40 (SV40), a mouse mammary tumor virus (MMTV) promoter, a human immunodeficiency virus (HIV) promoter such as the bovine immunodeficiency virus (BIV) long terminal repeat (LTR) promoter, a Moloney virus promoter, an avian leukosis virus (ALV) promoter, a cytomegalovirus (CMV) promoter such as the CMV immediate early promoter, Epstein Barr virus (EBV) promoter, or a Rous sarcoma virus (RSV) promoter. The promoter can also be a promoter from a human gene such as human ubiquitin C (hUbC), human actin, human myosin, human hemoglobin, human muscle creatine, or human metalothionein. The promoter can also be a tissue specific promoter, such as a muscle or skin specific promoter, natural or synthetic. Examples of such promoters are described in US Patent Application Publication No. US20040175727.

The vector can also comprise a polyadenylation signal, which can be downstream of the CRISPR/Cas9-based system. The polyadenylation signal can be a SV40 polyadenylation signal, LTR polyadenylation signal, bovine growth hormone (bGH) polyadenylation signal, human growth hormone (hGH) polyadenylation signal, or human β- globin polyadenylation signal. The SV40 polyadenylation signal can be a polyadenylation signal from a pCEP4 vector (Invitrogen, San Diego, CA).

The vector can also comprise an enhancer upstream of the CRISPR/Cas9-based system for DNA expression. The enhancer can be human actin, human myosin, human hemoglobin, human muscle creatine or a viral enhancer such as one from CMV, HA, RSV or EBV. Polynucleotide function enhancers are described in U.S. Patent Nos. 5,593,972, 5,962,428, and WO94/016737. The vector can also comprise a mammalian origin of replication in order to maintain the vector extrachromosomally and produce multiple copies of the vector in a cell. The vector can also comprise a regulatory sequence, which may be well suited for gene expression in a mammalian or human cell into which the vector is administered. The vector can also comprise a reporter gene, such as green fluorescent protein ("GFP") and/or a selectable marker, such as hygromycin ("Hygro").

The vectors can be expression vectors or systems to produce protein by routine techniques and readily available starting materials including Sambrook et al., Molecular Cloning and Laboratory Manual, Second Ed., Cold Spring Harbor (1989).

The presently disclosed vectors can be used for genome editing a *dystrophin* gene in skeletal muscle or cardiac muscle of a subject. The presently disclosed vectors can be used in correcting or reducing the effects of mutations in the *dystrophin* gene involved in genetic diseases and/or other skeletal or cardiac muscle conditions, e.g., DMD.

### 2.1 Dystrophin

Dystrophin is a rod-shaped cytoplasmic protein which is a part of a protein complex that connects the cytoskeleton of a muscle fiber to the surrounding extracellular matrix through the cell membrane. Dystrophin provides structural stability to the dystroglycan complex of the cell membrane. The dystrophin gene is 2.2 megabases at locus Xp21. The primary transcription measures about 2,400 kb with the mature m NA being about 14 kb. 79 exons code for the protein which is over 3500 amino acids. Normal skeleton muscle tissue contains only small amounts of dystrophin but its absence of abnormal expression leads to the development of severe and incurable symptoms. Some mutations in the dystrophin gene lead to the production of defective dystrophin and severe dystrophic phenotype in affected patients. Some mutations in the dystrophin gene lead to partially-functional dystrophin protein and a much milder dystrophic phenotype in affected patients.

In certain embodiments, a functional gene refers to a gene transcribed to mRNA, which is translated to a functional protein.

In certain embodiments, a "partially-functional" protein refers to a protein that is encoded by a mutant gene (e.g., a mutant *dystrophin* gene) and has less biological activity than a functional protein but more than a non-functional protein.

DMD is the result of inherited or spontaneous mutations that cause nonsense or frame shift mutations in the *dystrophin* gene. Naturally occurring mutations and their consequences are relatively well understood for DMD. It is known that in-frame deletions that occur in the exon 45-55 region (e.g., exon 51) contained within the rod domain can produce highly functional dystrophin proteins, and many carriers are asymptomatic or display mild symptoms. Furthermore, more than 60% of patients may theoretically be treated by targeting exon(s) in this region of the *dystrophin* gene (e.g., targeting exon 51). Efforts have been made to restore the disrupted *dystrophin* reading frame in DMD patients by skipping non-essential exon(s) (e.g., exon 51 skipping) during mRNA splicing to produce internally deleted but functional dystrophin proteins. The deletion of internal *dystrophin* exon(s) (e.g., deletion of exon 51) retains the proper reading frame but cause the less severe Becker muscular dystrophy.

In certain embodiments, modification of exon 51 (e.g., deletion or excision of exon 51 by, e.g., NHEJ) to restore reading frame ameliorates the phenotype of up to 17% of DMD subjects, and up to 21% of DMD subjects with deletion mutations (Flanigan et al., Human Mutation 2009; 30:1657-1666. Aartsma-Rus et al., Human Mutation 2009; 30:293-299. Bladen et al., Human Mutation 2015; 36(2)).

In certain embodiments, exon 51 of a *dystrophin* gene refers to the 51^{st} exon of the *dystrophin* gene. Exon 51 is frequently adjacent to frame-disrupting deletions in DMD patients and has been targeted in clinical trials for oligonucleotide-based exon skipping. A clinical trial for the exon 51 skipping compound eteplirsen reported a significant functional benefit across 48 weeks, with an average of 47% dystrophin positive fibers compared to baseline. Mutations in exon 51 are ideally suited for permanent correction by NHEJ-based genome editing.

### 2.2. CRISPR/Cas System Specific for a Dystrophin Gene

The presently disclosed vector encodes a CRISPR/Cas system that is specific for a *dystrophin* gene (e.g., human *dystrophin* gene). "Clustered Regularly Interspaced Short Palindromic Repeats" and "CRISPRs", as used interchangeably herein refers to loci containing multiple short direct repeats that are found in the genomes of approximately 40% of sequenced bacteria and 90% of sequenced archaea. The CRISPR system is a microbial nuclease system involved in defense against invading phages and plasmids that provides a form of acquired immunity. The CRISPR loci in microbial hosts contain a combination of CRISPR-associated (Cas) genes as well as non-coding RNA elements capable of programming the specificity of the CRISPR-mediated nucleic acid cleavage. Short segments of foreign DNA, called spacers, are incorporated into the genome between CRISPR repeats, and serve as a 'memory' of past exposures. Cas9 forms a complex with the 3' end of the sgRNA, and the protein-RNA pair recognizes its genomic target by complementary base pairing between the 5' end of the sgRNA sequence and a predefined 20 bp DNA sequence, known as the protospacer. This complex is directed to homologous loci of pathogen DNA via regions encoded within the crRNA, i.e., the protospacers, and protospacer-adjacent motifs (PAMs) within the pathogen genome. The non-coding CRISPR array is transcribed and cleaved within direct repeats into short crRNAs containing individual spacer sequences, which direct Cas nucleases to the target site (protospacer). By simply exchanging the 20 bp recognition sequence of the expressed sgRNA, the Cas9 nuclease can be directed to new genomic targets. CRISPR spacers are used to recognize and silence exogenous genetic elements in a manner analogous to RNAi in eukaryotic organisms.

In certain embodiments, complementarity refers to a property shared between two nucleic acid sequences, such that when they are aligned antiparallel to each other, the nucleotide bases at each position will be complementary.

Three classes of CRISPR systems (Types I, II and III effector systems) are known. The Type II effector system carries out targeted DNA double-strand break in four sequential steps, using a single effector enzyme, Cas9, to cleave dsDNA. Compared to the Type I and Type III effector systems, which require multiple distinct effectors acting as a complex, the Type II effector system may function in alternative contexts such as eukaryotic cells. The Type II effector system consists of a long pre-crRNA, which is transcribed from the spacer-containing CRISPR locus, the Cas9 protein, and a tracrRNA, which is involved in pre-crRNA processing. The tracrRNAs hybridize to the repeat regions separating the spacers of the pre-crRNA, thus initiating dsRNA cleavage by endogenous RNase III. This cleavage is followed by a second cleavage event within each spacer by Cas9, producing mature crRNAs that remain associated with the tracrRNA and Cas9, forming a Cas9:crRNA-tracrRNA complex.

The Cas9:crRNA-tracrRNA complex unwinds the DNA duplex and searches for sequences matching the crRNA to cleave. Target recognition occurs upon detection of complementarity between a "protospacer" sequence in the target DNA and the remaining spacer sequence in the crRNA. Cas9 mediates cleavage of target DNA if a correct protospacer-adjacent motif (PAM) is also present at the 3' end of the protospacer. For protospacer targeting, the sequence must be immediately followed by the protospacer-adjacent motif (PAM), a short sequence recognized by the Cas9 nuclease that is required for DNA cleavage. Different Type II systems have differing PAM requirements. The *S. pyogenes* CRISPR system may have the PAM sequence for this Cas9 (SpCas9) as 5'-NRG-3', where R is either A or G, and characterized the specificity of this system in human cells. A unique capability of the CRISPR/Cas9 system is the straightforward ability to simultaneously target multiple distinct genomic loci by co-expressing a single Cas9 protein with two or more gRNAs. For example, the *Streptococcus pyogenes* Type II system naturally prefers to use an "NGG" sequence, where "N" can be any nucleotide, but also accepts other PAM sequences, such as "NAG" in engineered systems (Hsu et al, Nature Biotechnology (2013) doi: 10.1038/nbt.2647). Similarly, the Cas9 derived from *Neisseria meningitidis* (NmCas9) normally has a native PAM of NNNNGATT (SEQ ID NO: 17), but has activity across a variety of PAMs, including a highly degenerate NNNNGNNN (SEQ ID NO: 18) PAM (Esvelt et al. Nature Methods (2013) doi: 10.1038/nmeth.2681). A Cas9 molecule of *S. aureus* recognizes the sequence motif NNGRR (R = A or G) (SEQ ID NO: 22) and directs cleavage of a target nucleic acid sequence 1 to 10, e.g., 3 to 5, bp upstream from that sequence. A Cas9 molecule of *S. aureus* recognizes the sequence motif NNGRRN (R = A or G) (SEQ ID NO: 23) and directs cleavage of a target nucleic acid sequence 1 to 10, e.g., 3 to 5, bp upstream from that sequence. In certain embodiments, a Cas9 molecule of *S. aureus* recognizes the sequence motif NNGRRT (R = A or G) (SEQ ID NO: 24) and directs cleavage of a target nucleic acid sequence 1 to 10, e.g., 3 to 5, bp upstream from that sequence. In certain embodiments, a Cas9 molecule of *S. aureus* recognizes the sequence motif NNGRRV (R = A or G) (SEQ ID NO: 25) and directs cleavage of a target nucleic acid sequence 1 to 10, e.g., 3 to 5, bp upstream from that sequence. In the aforementioned embodiments, N can be any nucleotide residue, e.g., any of A, G, C, or T. Cas9 molecules can be engineered to alter the PAM specificity of the Cas9 molecule.

An engineered form of the Type II effector system of *Streptococcus pyogenes* was shown to function in human cells for genome engineering. In this system, the Cas9 protein was directed to genomic target sites by a synthetically reconstituted "guide RNA" ("gRNA", also used interchangeably herein as a chimeric single guide RNA ("sgRNA")), which is a crRNA-tracrRNA fusion that obviates the need for RNase III and crRNA processing in general. Provided herein are CRISPR/Cas9-based engineered systems for use in genome editing and treating genetic diseases. The CRISPR/Cas9-based engineered systems can be designed to target any gene, including genes involved in a genetic disease, aging, tissue regeneration, or wound healing. The CRISPR/Cas9-based systems can include a Cas9 protein and at least one gRNA. The system comprises two gRNA molecules. The Cas9 protein may be part of a Cas9 fusion protein. The vector may further encode a Cas9 fusion protein. The Cas9 fusion protein may, for example, include a domain that has a different activity that what is endogenous to Cas9, such as a transactivation domain. The target gene (e.g., a *dystrophin* gene, e.g., human *dystrophin* gene) can be involved in differentiation of a cell or any other process in which activation of a gene can be desired, or can have a mutation such as a frameshift mutation or a nonsense mutation. If the target gene has a mutation that causes a premature stop codon, an aberrant splice acceptor site or an aberrant splice donor site, the CRISPR/Cas9-based system can be designed to recognize and bind a nucleotide sequence upstream or downstream from the premature stop codon, the aberrant splice acceptor site or the aberrant splice donor site. The CRISPR-Cas9-based system can also be used to disrupt normal gene splicing by targeting splice acceptors and donors to induce skipping of premature stop codons or restore a disrupted reading frame. The CRISPR/Cas9-based system may or may not mediate off-target changes to protein-coding regions of the genome.

### 2.2.1 Cas9 molecules and Cas9 fusion proteins

The CRISPR/Cas9-based system can include a Cas9 protein or a Cas9 fusion protein. Cas9 protein is an endonuclease that cleaves nucleic acid and is encoded by the CRISPR loci and is involved in the Type II CRISPR system. In the present disclosure, the Cas9 molecule is a *Staphylococcus aureus* Cas9 molecule.

Alternatively or additionally, the CRISPR/Cas9-based system can include a fusion protein. The fusion protein can comprise two heterologous polypeptide domains, wherein the first polypeptide domain comprises a Cas protein and the second polypeptide domain has an activity such as transcription activation activity, transcription repression activity, transcription release factor activity, histone modification activity, nuclease activity, nucleic acid association activity, methylase activity, or demethylase activity. The fusion protein can include a Cas9 protein or a mutated Cas9 protein, fused to a second polypeptide domain that has an activity such as transcription activation activity, transcription repression activity, transcription release factor activity, histone modification activity, nuclease activity, nucleic acid association activity, methylase activity, or demethylase activity.

### (1) Transcription Activation Activity

The second polypeptide domain can have transcription activation activity, i.e., a transactivation domain. For example, gene expression of endogenous mammalian genes, such as human genes, can be achieved by targeting a fusion protein of iCas9 and a transactivation domain to mammalian promoters via combinations of gRNAs. The transactivation domain can include a VP 16 protein, multiple VP 16 proteins, such as a VP48 domain or VP64 domain, or p65 domain of NF kappa B transcription activator activity. For example, the fusion protein may be iCas9-VP64.

### (2) Transcription Repression Activity

The second polypeptide domain can have transcription repression activity. The second polypeptide domain can have a Kruppel associated box activity, such as a KRAB domain, ERF repressor domain activity, Mxil repressor domain activity, SID4X repressor domain activity, Mad-SID repressor domain activity or TATA box binding protein activity. For example, the fusion protein may be dCas9-KRAB.

### (3) Transcription Release Factor Activity

The second polypeptide domain can have transcription release factor activity. The second polypeptide domain can have eukaryotic release factor 1 (ERF1) activity or eukaryotic release factor 3 (ERF3) activity.

### (4) Histone Modification Activity

The second polypeptide domain can have histone modification activity. The second polypeptide domain can have histone deacetylase, histone acetyltransferase, histone demethylase, or histone methyltransferase activity. The histone acetyltransferase may be p300 or CREB-binding protein (CBP) protein, or fragments thereof. For example, the fusion protein may be dCas9-p300.

### (5) Nuclease Activity

The second polypeptide domain can have nuclease activity that is different from the nuclease activity of the Cas9 protein. A nuclease, or a protein having nuclease activity, is an enzyme capable of cleaving the phosphodiester bonds between the nucleotide subunits of nucleic acids. Nucleases are usually further divided into endonucleases and exonucleases, although some of the enzymes may fall in both categories. Well known nucleases are deoxyribonuclease and ribonuclease.

### (6) Nucleic Acid Association Activity

The second polypeptide domain can have nucleic acid association activity or nucleic acid binding protein-DNA-binding domain (DBD) is an independently folded protein domain that contains at least one motif that recognizes double- or single-stranded DNA. A DBD can recognize a specific DNA sequence (a recognition sequence) or have a general affinity to DNA. nucleic acid association region selected from the group consisting of helix-turn-helix region, leucine zipper region, winged helix region, winged helix-turn-helix region, helix-loop-helix region, immunoglobulin fold, B3 domain, Zinc finger, HMG-box, Wor3 domain, TAL effector DNA-binding domain.

### (7) Methylase Activity

The second polypeptide domain can have methylase activity, which involves transferring a methyl group to DNA, RNA, protein, small molecule, cytosine or adenine. The second polypeptide domain may include a DNA methyltransferase.

### (8) Demethylase Activity

The second polypeptide domain can have demethylase activity. The second polypeptide domain can include an enzyme that remove methyl (CH3-) groups from nucleic acids, proteins (in particular histones), and other molecules. Alternatively, the second polypeptide can covert the methyl group to hydroxymethylcytosine in a mechanism for demethylating DNA. The second polypeptide can catalyze this reaction. For example, the second polypeptide that catalyzes this reaction can be Tet1.

A Cas9 molecule or a Cas9 fusion protein can interact with one or more gRNA molecule and, in concert with the gRNA molecule(s), localizes to a site which comprises a target domain, and a PAM sequence. The ability of a Cas9 molecule or a Cas9 fusion protein to recognize a PAM sequence can be determined, e.g., using a transformation assay as described previously (Jinek 2012).

The ability of a Cas9 molecule or a Cas9 fusion protein to interact with and cleave a target nucleic acid is PAM sequence dependent. A PAM sequence is a sequence in the target nucleic acid. In certain embodiments, cleavage of the target nucleic acid occurs upstream from the PAM sequence. Cas9 molecules from different bacterial species can recognize different sequence motifs (e.g., PAM sequences). A Cas9 molecule of *S. pyogenes* recognizes the sequence motif NGG and directs cleavage of a target nucleic acid sequence 1 to 10, e.g., 3 to 5, bp upstream from that sequence (see, e.g., Mali 2013). A Cas9 molecule of *S. thermophilus* recognizes the sequence motif NGGNG (SEQ ID NO: 19) and/or NNAGAAW (W = A or T) (SEQ ID NO: 20) and directs cleavage of a target nucleic acid sequence 1 to 10, e.g., 3 to 5, bp upstream from these sequences (see, e.g., Horvath 2010; Deveau 2008). A Cas9 molecule of *S*. *mutans* can recognize the sequence motif NGG and/or NAAR (R = A or G) (SEQ ID NO: 21) and direct cleavage of a target nucleic acid sequence 1 to 10, e.g., 3 to 5 bp, upstream from this sequence (see, e.g., Deveau 2008). A Cas9 molecule of *S. aureus* recognizes the sequence motif NNGRR (R = A or G) (SEQ ID NO: 22) and directs cleavage of a target nucleic acid sequence 1 to 10, e.g., 3 to 5, bp upstream from that sequence. A Cas9 molecule of *S. aureus* recognizes the sequence motif NNGRRN (R = A or G) (SEQ ID NO: 23) and directs cleavage of a target nucleic acid sequence 1 to 10, e.g., 3 to 5, bp upstream from that sequence. A Cas9 molecule of *S. aureus* recognizes the sequence motif NNGRRT (R = A or G) (SEQ ID NO: 24) and directs cleavage of a target nucleic acid sequence 1 to 10, e.g., 3 to 5, bp upstream from that sequenceA Cas9 molecule of *S. aureus* recognizes the sequence motif NNGRRV (R = A or G) (SEQ ID NO: 25) and directs cleavage of a target nucleic acid sequence 1 to 10, e.g., 3 to 5, bp upstream from that sequence. In the aforementioned disclosure, N can be any nucleotide residue, e.g., any of A, G, C, or T. Cas9 molecules can be engineered to alter the PAM specificity of the Cas9 molecule.

In the present disclosure, the vector encodes at least one Cas9 molecule that recognizes a Protospacer Adjacent Motif (PAM) of either NNGRRT (SEQ ID NO: 24) or NNGRRV (SEQ ID NO: 25). The at least one Cas9 molecule is an *S. aureus* Cas9 molecule. In certain embodiments, the at least one Cas9 molecule is a mutant *S. aureus* Cas9 molecule.

The Cas9 protein can be mutated so that the nuclease activity is inactivated. An inactivated Cas9 protein ("iCas9", also referred to as "dCas9") with no endonuclease activity has been recently targeted to genes in bacteria, yeast, and human cells by gRNAs to silence gene expression through steric hindrance. Exemplary mutations with reference to the *S. pyogenes* Cas9 sequence include: D10A, E762A, H840A, N854A, N863A and/or D986A. Exemplary mutations with reference to the *S. aureus* Cas9 sequence include D10A and N580A. In certain embodiments, the Cas9 molecule is a mutant *S. aureus* Cas9 molecule. In certain embodiments, the mutant *S. aureus* Cas9 molecule comprises a D10A mutation. The nucleotide sequence encoding this mutant *S. aureus* Cas9 is set forth in SEQ ID NO: 34, which is provided below.

atgaaaagga actacattct ggggctggcc atcgggatta caagcgtggg gtatgggatt
attgactatg aaacaaggga cgtgatcgac gcaggcgtca gactgttcaa ggaggccaac
gtggaaaaca atgagggacg gagaagcaag aggggagcca ggcgcctgaa acgacggaga
aggcacagaa tccagagggt gaagaaactg ctgttcgatt acaacctgct gaccgaccat
tctgagctga gtggaattaa tccttatgaa gccagggtga aaggcctgag tcagaagctg
tcagaggaag agttttccgc agctctgctg cacctggcta agcgccgagg agtgcataac
gtcaatgagg tggaagagga caccggcaac gagctgtcta caaaggaaca gatctcacgc
aatagcaaag ctctggaaga gaagtatgtc gcagagctgc agctggaacg gctgaagaaa
gatggcgagg tgagagggtc aattaatagg ttcaagacaa gcgactacgt caaagaagcc
aagcagctgc tgaaagtgca gaaggcttac caccagctgg atcagagctt catcgatact
tatatcgacc tgctggagac tcggagaacc tactatgagg gaccaggaga agggagcccc
ttcggatgga aagacatcaa ggaatggtac gagatgctga tgggacattg cacctatttt
ccagaagagc tgagaagcgt caagtacgct tataacgcag atctgtacaa cgccctgaat
gacctgaaca acctggtcat caccagggat gaaaacgaga aactggaata ctatgagaag
ttccagatca tcgaaaacgt gtttaagcag aagaaaaagc ctacactgaa acagattgct
aaggagatcc tggtcaacga agaggacatc aagggctacc gggtgacaag cactggaaaa
ccagagttca ccaatctgaa agtgtatcac gatattaagg acatcacagc acggaaagaa
atcattgaga acgccgaact gctggatcag attgctaaga tcctgactat ctaccagagc
tccgaggaca tccaggaaga gctgactaac ctgaacagcg agctgaccca ggaagagatc
gaacagatta gtaatctgaa ggggtacacc ggaacacaca acctgtccct gaaagctatc
aatctgattc tggatgagct gtggcataca aacgacaatc agattgcaat ctttaaccgg
ctgaagctgg tcccaaaaaa ggtggacctg agtcagcaga aagagatccc aaccacactg
gtggacgatt tcattctgtc acccgtggtc aagcggagct tcatccagag catcaaagtg
atcaacgcca tcatcaagaa gtacggcctg cccaatgata tcattatcga gctggctagg
gagaagaaca gcaaggacgc acagaagatg atcaatgaga tgcagaaacg aaaccggcag
accaatgaac gcattgaaga gattatccga actaccggga aagagaacgc aaagtacctg
attgaaaaaa tcaagctgca cgatatgcag gagggaaagt gtctgtattc tctggaggcc
atccccctgg aggacctgct gaacaatcca ttcaactacg aggtcgatca tattatcccc
agaagcgtgt ccttcgacaa ttcctttaac aacaaggtgc tggtcaagca ggaagagaac
tctaaaaagg gcaataggac tcctttccag tacctgtcta gttcagattc caagatctct
tacgaaacct ttaaaaagca cattctgaat ctggccaaag gaaagggccg catcagcaag
accaaaaagg agtacctgct ggaagagcgg gacatcaaca gattctccgt ccagaaggat
tttattaacc ggaatctggt ggacacaaga tacgctactc gcggcctgat gaatctgctg
cgatcctatt tccgggtgaa caatctggat gtgaaagtca agtccatcaa cggcgggttc
acatcttttc tgaggcgcaa atggaagttt aaaaaggagc gcaacaaagg gtacaagcac
catgccgaag atgctctgat tatcgcaaat gccgacttca tctttaagga gtggaaaaag
ctggacaaag ccaagaaagt gatggagaac cagatgttcg aagagaagca ggccgaatct
atgcccgaaa tcgagacaga acaggagtac aaggagattt tcatcactcc tcaccagatc
aagcatatca aggatttcaa ggactacaag tactctcacc gggtggataa aaagcccaac
agagagctga tcaatgacac cctgtatagt acaagaaaag acgataaggg gaataccctg
attgtgaaca atctgaacgg actgtacgac aaagataatg acaagctgaa aaagctgatc
aacaaaagtc ccgagaagct gctgatgtac caccatgatc ctcagacata tcagaaactg
aagctgatta tggagcagta cggcgacgag aagaacccac tgtataagta ctatgaagag
actgggaact acctgaccaa gtatagcaaa aaggataatg gccccgtgat caagaagatc

aagtactatg ggaacaagct gaatgcccat ctggacatca cagacgatta ccctaacagt
cgcaacaagg tggtcaagct gtcactgaag ccatacagat tcgatgtcta tctggacaac
ggcgtgtata aatttgtgac tgtcaagaat ctggatgtca tcaaaaagga gaactactat
gaagtgaata gcaagtgcta cgaagaggct aaaaagctga aaaagattag caaccaggca
gagttcatcg cctcctttta caacaacgac ctgattaaga tcaatggcga actgtatagg
gtcatcgggg tgaacaatga tctgctgaac cgcattgaag tgaatatgat tgacatcact
taccgagagt atctggaaaa catgaatgat aagcgccccc ctcgaattat caaaacaatt
gcctctaaga ctcagagtat caaaaagtac tcaaccgaca ttctgggaaa cctgtatgag
gtgaagagca aaaagcaccc tcagattatc aaaaagggc [SEQ ID NO: 34]

In certain embodiments, the mutant *S. aureus* Cas9 molecule comprises a N580A mutation. The nucleotide sequence encoding this mutant *S. aureus* Cas9 molecule is set forth in SEQ ID NO: 35, which is provided below.

atgaaaagga actacattct ggggctggac atcgggatta caagcgtggg gtatgggatt
attgactatg aaacaaggga cgtgatcgac gcaggcgtca gactgttcaa ggaggccaac
gtggaaaaca atgagggacg gagaagcaag aggggagcca ggcgcctgaa acgacggaga
aggcacagaa tccagagggt gaagaaactg ctgttcgatt acaacctgct gaccgaccat
tctgagctga gtggaattaa tccttatgaa gccagggtga aaggcctgag tcagaagctg
tcagaggaag agttttccgc agctctgctg cacctggcta agcgccgagg agtgcataac
gtcaatgagg tggaagagga caccggcaac gagctgtcta caaaggaaca gatctcacgc
aatagcaaag ctctggaaga gaagtatgtc gcagagctgc agctggaacg gctgaagaaa
gatggcgagg tgagagggtc aattaatagg ttcaagacaa gcgactacgt caaagaagcc
aagcagctgc tgaaagtgca gaaggcttac caccagctgg atcagagctt catcgatact
tatatcgacc tgctggagac tcggagaacc tactatgagg gaccaggaga agggagcccc
ttcggatgga aagacatcaa ggaatggtac gagatgctga tgggacattg cacctatttt
ccagaagagc tgagaagcgt caagtacgct tataacgcag atctgtacaa cgccctgaat
gacctgaaca acctggtcat caccagggat gaaaacgaga aactggaata ctatgagaag
ttccagatca tcgaaaacgt gtttaagcag aagaaaaagc ctacactgaa acagattgct
aaggagatcc tggtcaacga agaggacatc aagggctacc gggtgacaag cactggaaaa
ccagagttca ccaatctgaa agtgtatcac gatattaagg acatcacagc acggaaagaa
atcattgaga acgccgaact gctggatcag attgctaaga tcctgactat ctaccagagc
tccgaggaca tccaggaaga gctgactaac ctgaacagcg agctgaccca ggaagagatc
gaacagatta gtaatctgaa ggggtacacc ggaacacaca acctgtccct gaaagctatc
aatctgattc tggatgagct gtggcataca aacgacaatc agattgcaat ctttaaccgg
ctgaagctgg tcccaaaaaa ggtggacctg agtcagcaga aagagatccc aaccacactg
gtggacgatt tcattctgtc acccgtggtc aagcggagct tcatccagag catcaaagtg
atcaacgcca tcatcaagaa gtacggcctg cccaatgata tcattatcga gctggctagg
gagaagaaca gcaaggacgc acagaagatg atcaatgaga tgcagaaacg aaaccggcag
accaatgaac gcattgaaga gattatccga actaccggga aagagaacgc aaagtacctg
attgaaaaaa tcaagctgca cgatatgcag gagggaaagt gtctgtattc tctggaggcc
atccccctgg aggacctgct gaacaatcca ttcaactacg aggtcgatca tattatcccc
agaagcgtgt ccttcgacaa ttcctttaac aacaaggtgc tggtcaagca ggaagaggcc
tctaaaaagg gcaataggac tcctttccag tacctgtcta gttcagattc caagatctct
tacgaaacct ttaaaaagca cattctgaat ctggccaaag gaaagggccg catcagcaag
accaaaaagg agtacctgct ggaagagcgg gacatcaaca gattctccgt ccagaaggat
tttattaacc ggaatctggt ggacacaaga tacgctactc gcggcctgat gaatctgctg
cgatcctatt tccgggtgaa caatctggat gtgaaagtca agtccatcaa cggcgggttc
acatcttttc tgaggcgcaa atggaagttt aaaaaggagc gcaacaaagg gtacaagcac
catgccgaag atgctctgat tatcgcaaat gccgacttca tctttaagga gtggaaaaag
ctggacaaag ccaagaaagt gatggagaac cagatgttcg aagagaagca ggccgaatct
atgcccgaaa tcgagacaga acaggagtac aaggagattt tcatcactcc tcaccagatc
aagcatatca aggatttcaa ggactacaag tactctcacc gggtggataa aaagcccaac
agagagctga tcaatgacac cctgtatagt acaagaaaag acgataaggg gaataccctg

attgtgaaca atctgaacgg actgtacgac aaagataatg acaagctgaa aaagctgatc
aacaaaagtc ccgagaagct gctgatgtac caccatgatc ctcagacata tcagaaactg
aagctgatta tggagcagta cggcgacgag aagaacccac tgtataagta ctatgaagag
actgggaact acctgaccaa gtatagcaaa aaggataatg gccccgtgat caagaagatc
aagtactatg ggaacaagct gaatgcccat ctggacatca cagacgatta ccctaacagt
cgcaacaagg tggtcaagct gtcactgaag ccatacagat tcgatgtcta tctggacaac
ggcgtgtata aatttgtgac tgtcaagaat ctggatgtca tcaaaaagga gaactactat
gaagtgaata gcaagtgcta cgaagaggct aaaaagctga aaaagattag caaccaggca
gagttcatcg cctcctttta caacaacgac ctgattaaga tcaatggcga actgtatagg
gtcatcgggg tgaacaatga tctgctgaac cgcattgaag tgaatatgat tgacatcact
taccgagagt atctggaaaa catgaatgat aagcgccccc ctcgaattat caaaacaatt
gcctctaaga ctcagagtat caaaaagtac tcaaccgaca ttctgggaaa cctgtatgag
gtgaagagca aaaagcaccc tcagattatc aaaaagggc [SEQ ID NO: 35]

A nucleic acid encoding a Cas9 molecule can be a synthetic nucleic acid sequence. For example, the synthetic nucleic acid molecule can be chemically modified. The synthetic nucleic acid sequence can be codon optimized, e.g., at least one non-common codon or less-common codon has been replaced by a common codon. For example, the synthetic nucleic acid can direct the synthesis of an optimized messenger mRNA, e.g., optimized for expression in a mammalian expression system, e.g., described herein.

Additionally or alternatively, a nucleic acid encoding a Cas9 molecule or Cas9 polypeptide may comprise a nuclear localization sequence (NLS). Nuclear localization sequences are known in the art.

An exemplary codon optimized nucleic acid sequence encoding a Cas9 molecule of *S*. *pyogenes* is set forth in SEQ ID NO: 26, which is provided below.

atggataaaa agtacagcat cgggctggac atcggtacaa actcagtggg gtgggccgtg
attacggacg agtacaaggt accctccaaa aaatttaaag tgctgggtaa cacggacaga
cactctataa agaaaaatct tattggagcc ttgctgttcg actcaggcga gacagccgaa
gccacaaggt tgaagcggac cgccaggagg cggtatacca ggagaaagaa ccgcatatgc
tacctgcaag aaatcttcag taacgagatg gcaaaggttg acgatagctt tttccatcgc
ctggaagaat cctttcttgt tgaggaagac aagaagcacg aacggcaccc catctttggc
aatattgtcg acgaagtggc atatcacgaa aagtacccga ctatctacca cctcaggaag
aagctggtgg actctaccga taaggcggac ctcagactta tttatttggc actcgcccac
atgattaaat ttagaggaca tttcttgatc gagggcgacc tgaacccgga caacagtgac
gtcgataagc tgttcatcca acttgtgcag acctacaatc aactgttcga agaaaaccct
ataaatgctt caggagtcga cgctaaagca atcctgtccg cgcgcctctc aaaatctaga
agacttgaga atctgattgc tcagttgccc ggggaaaaga aaaatggatt gtttggcaac
ctgatcgccc tcagtctcgg actgacccca aatttcaaaa gtaacttcga cctggccgaa
gacgctaagc tccagctgtc caaggacaca tacgatgacg acctcgacaa tctgctggcc
cagattgggg atcagtacgc cgatctcttt ttggcagcaa agaacctgtc cgacgccatc
ctgttgagcg atatcttgag agtgaacacc gaaattacta aagcacccct tagcgcatct
atgatcaagc ggtacgacga gcatcatcag gatctgaccc tgctgaaggc tcttgtgagg
caacagctcc ccgaaaaata caaggaaatc ttctttgacc agagcaaaaa cggctacgct
ggctatatag atggtggggc cagtcaggag gaattctata aattcatcaa gcccattctc
gagaaaatgg acggcacaga ggagttgctg gtcaaactta acagggagga cctgctgcgg
aagcagcgga cctttgacaa cgggtctatc ccccaccaga ttcatctggg cgaactgcac
gcaatcctga ggaggcagga ggatttttat cctttcttta aagataaccg cgagaaaata
gaaaagattc ttacattcag gatcccgtac tacgtgggac ctctcgcccg gggcaattca

cggtttgcct ggatgacaag gaagtcagag gagactatta caccttggaa cttcgaagaa
gtggtggaca agggtgcatc tgcccagtct ttcatcgagc ggatgacaaa ttttgacaag
aacctcccta atgagaaggt gctgcccaaa cattctctgc tctacgagta ctttaccgtc
tacaatgaac tgactaaagt caagtacgtc accgagggaa tgaggaagcc ggcattcctt
agtggagaac agaagaaggc gattgtagac ctgttgttca agaccaacag gaaggtgact
gtgaagcaac ttaaagaaga ctactttaag aagatcgaat gttttgacag tgtggaaatt
tcaggggttg aagaccgctt caatgcgtca ttggggactt accatgatct tctcaagatc
ataaaggaca aagacttcct ggacaacgaa gaaaatgagg atattctcga agacatcgtc
ctcaccctga ccctgttcga agacagggaa atgatagaag agcgcttgaa aacctatgcc
cacctcttcg acgataaagt tatgaagcag ctgaagcgca ggagatacac aggatgggga
agattgtcaa ggaagctgat caatggaatt agggataaac agagtggcaa gaccatactg
gatttcctca aatctgatgg cttcgccaat aggaacttca tgcaactgat tcacgatgac
tctcttacct tcaaggagga cattcaaaag gctcaggtga gcgggcaggg agactccctt
catgaacaca tcgcgaattt ggcaggttcc cccgctatta aaaagggcat ccttcaaact
gtcaaggtgg tggatgaatt ggtcaaggta atgggcagac ataagccaga aaatattgtg
atcgagatgg cccgcgaaaa ccagaccaca cagaagggcc agaaaaatag tagagagcgg
atgaagagga tcgaggaggg catcaaagag ctgggatctc agattctcaa agaacacccc
gtagaaaaca cacagctgca gaacgaaaaa ttgtacttgt actatctgca gaacggcaga
gacatgtacg tcgaccaaga acttgatatt aatagactgt ccgactatga cgtagaccat
atcgtgcccc agtccttcct gaaggacgac tccattgata acaaagtctt gacaagaagc
gacaagaaca ggggtaaaag tgataatgtg cctagcgagg aggtggtgaa aaaaatgaag
aactactggc gacagctgct taatgcaaag ctcattacac aacggaagtt cgataatctg
acgaaagcag agagaggtgg cttgtctgag ttggacaagg cagggtttat taagcggcag
ctggtggaaa ctaggcagat cacaaagcac gtggcgcaga ttttggacag ccggatgaac
acaaaatacg acgaaaatga taaactgata cgagaggtca aagttatcac gctgaaaagc
aagctggtgt ccgattttcg gaaagacttc cagttctaca aagttcgcga gattaataac
taccatcatg ctcacgatgc gtacctgaac gctgttgtcg ggaccgcctt gataaagaag
tacccaaagc tggaatccga gttcgtatac ggggattaca aagtgtacga tgtgaggaaa
atgatagcca agtccgagca ggagattgga aaggccacag ctaagtactt cttttattct
aacatcatga atttttttaa gacggaaatt accctggcca acggagagat cagaaagcgg
ccccttatag agacaaatgg tgaaacaggt gaaatcgtct gggataaggg cagggatttc
gctactgtga ggaaggtgct gagtatgcca caggtaaata tcgtgaaaaa aaccgaagta
cagaccggag gattttccaa ggaaagcatt ttgcctaaaa gaaactcaga caagctcatc
gcccgcaaga aagattggga ccctaagaaa tacgggggat ttgactcacc caccgtagcc
tattctgtgc tggtggtagc taaggtggaa aaaggaaagt ctaagaagct gaagtccgtg
aaggaactct tgggaatcac tatcatggaa agatcatcct ttgaaaagaa ccctatcgat
ttcctggagg ctaagggtta caaggaggtc aagaaagacc tcatcattaa actgccaaaa
tactctctct tcgagctgga aaatggcagg aagagaatgt tggccagcgc cggagagctg
caaaagggaa acgagcttgc tctgccctcc aaatatgtta attttctcta tctcgcttcc
cactatgaaa agctgaaagg gtctcccgaa gataacgagc agaagcagct gttcgtcgaa
cagcacaagc actatctgga tgaaataatc gaacaaataa gcgagttcag caaaagggtt
atcctggcgg atgctaattt ggacaaagta ctgtctgctt ataacaagca ccgggataag
cctattaggg aacaagccga gaatataatt cacctcttta cactcacgaa tctcggagcc
cccgccgcct tcaaatactt tgatacgact atcgaccgga aacggtatac cagtaccaaa
gaggtcctcg atgccaccct catccaccag tcaattactg gcctgtacga aacacggatc
gacctctctc aactgggcgg cgactag [SEQ ID NO: 26]

The corresponding amino acid sequence of an *S. pyogenes* Cas9 molecule is set forth in SEQ ID NO: 27, which is provided below.
























 [SEQ ID NO: 27]

Exemplary codon optimized nucleic acid sequences encoding a Cas9 molecule of *S. aureus* are set forth in SEQ ID NOs: 28-32, which are provided below.
SEQ ID NO: 28 is set forth below: 
atgaaaagga actacattct ggggctggac atcgggatta caagcgtggg gtatgggatt
attgactatg aaacaaggga cgtgatcgac gcaggcgtca gactgttcaa ggaggccaac
gtggaaaaca atgagggacg gagaagcaag aggggagcca ggcgcctgaa acgacggaga
aggcacagaa tccagagggt gaagaaactg ctgttcgatt acaacctgct gaccgaccat
tctgagctga gtggaattaa tccttatgaa gccagggtga aaggcctgag tcagaagctg
tcagaggaag agttttccgc agctctgctg cacctggcta agcgccgagg agtgcataac
gtcaatgagg tggaagagga caccggcaac gagctgtcta caaaggaaca gatctcacgc
aatagcaaag ctctggaaga gaagtatgtc gcagagctgc agctggaacg gctgaagaaa
gatggcgagg tgagagggtc aattaatagg ttcaagacaa gcgactacgt caaagaagcc
aagcagctgc tgaaagtgca gaaggcttac caccagctgg atcagagctt catcgatact
tatatcgacc tgctggagac tcggagaacc tactatgagg gaccaggaga agggagcccc
ttcggatgga aagacatcaa ggaatggtac gagatgctga tgggacattg cacctatttt
ccagaagagc tgagaagcgt caagtacgct tataacgcag atctgtacaa cgccctgaat
gacctgaaca acctggtcat caccagggat gaaaacgaga aactggaata ctatgagaag
ttccagatca tcgaaaacgt gtttaagcag aagaaaaagc ctacactgaa acagattgct
aaggagatcc tggtcaacga agaggacatc aagggctacc gggtgacaag cactggaaaa
ccagagttca ccaatctgaa agtgtatcac gatattaagg acatcacagc acggaaagaa
atcattgaga acgccgaact gctggatcag attgctaaga tcctgactat ctaccagagc
tccgaggaca tccaggaaga gctgactaac ctgaacagcg agctgaccca ggaagagatc
gaacagatta gtaatctgaa ggggtacacc ggaacacaca acctgtccct gaaagctatc
aatctgattc tggatgagct gtggcataca aacgacaatc agattgcaat ctttaaccgg
ctgaagctgg tcccaaaaaa ggtggacctg agtcagcaga aagagatccc aaccacactg
gtggacgatt tcattctgtc acccgtggtc aagcggagct tcatccagag catcaaagtg
atcaacgcca tcatcaagaa gtacggcctg cccaatgata tcattatcga gctggctagg
gagaagaaca gcaaggacgc acagaagatg atcaatgaga tgcagaaacg aaaccggcag
accaatgaac gcattgaaga gattatccga actaccggga aagagaacgc aaagtacctg
attgaaaaaa tcaagctgca cgatatgcag gagggaaagt gtctgtattc tctggaggcc
atccccctgg aggacctgct gaacaatcca ttcaactacg aggtcgatca tattatcccc
agaagcgtgt ccttcgacaa ttcctttaac aacaaggtgc tggtcaagca ggaagagaac
tctaaaaagg gcaataggac tcctttccag tacctgtcta gttcagattc caagatctct
tacgaaacct ttaaaaagca cattctgaat ctggccaaag gaaagggccg catcagcaag
accaaaaagg agtacctgct ggaagagcgg gacatcaaca gattctccgt ccagaaggat
tttattaacc ggaatctggt ggacacaaga tacgctactc gcggcctgat gaatctgctg
 
cgatcctatt tccgggtgaa caatctggat gtgaaagtca agtccatcaa cggcgggttc
acatcttttc tgaggcgcaa atggaagttt aaaaaggagc gcaacaaagg gtacaagcac
catgccgaag atgctctgat tatcgcaaat gccgacttca tctttaagga gtggaaaaag
ctggacaaag ccaagaaagt gatggagaac cagatgttcg aagagaagca ggccgaatct
atgcccgaaa tcgagacaga acaggagtac aaggagattt tcatcactcc tcaccagatc
aagcatatca aggatttcaa ggactacaag tactctcacc gggtggataa aaagcccaac
agagagctga tcaatgacac cctgtatagt acaagaaaag acgataaggg gaataccctg
attgtgaaca atctgaacgg actgtacgac aaagataatg acaagctgaa aaagctgatc
aacaaaagtc ccgagaagct gctgatgtac caccatgatc ctcagacata tcagaaactg
aagctgatta tggagcagta cggcgacgag aagaacccac tgtataagta ctatgaagag
actgggaact acctgaccaa gtatagcaaa aaggataatg gccccgtgat caagaagatc
aagtactatg ggaacaagct gaatgcccat ctggacatca cagacgatta ccctaacagt
cgcaacaagg tggtcaagct gtcactgaag ccatacagat tcgatgtcta tctggacaac
ggcgtgtata aatttgtgac tgtcaagaat ctggatgtca tcaaaaagga gaactactat
gaagtgaata gcaagtgcta cgaagaggct aaaaagctga aaaagattag caaccaggca
gagttcatcg cctcctttta caacaacgac ctgattaaga tcaatggcga actgtatagg
gtcatcgggg tgaacaatga tctgctgaac cgcattgaag tgaatatgat tgacatcact
taccgagagt atctggaaaa catgaatgat aagcgccccc ctcgaattat caaaacaatt
gcctctaaga ctcagagtat caaaaagtac tcaaccgaca ttctgggaaa cctgtatgag
gtgaagagca aaaagcaccc tcagattatc aaaaagggc [SEQ ID NO: 28]
SEQ ID NO: 29 is set forth below. 
atgaagcgga actacatcct gggcctggac atcggcatca ccagcgtggg ctacggcatc
atcgactacg agacacggga cgtgatcgat gccggcgtgc ggctgttcaa agaggccaac
gtggaaaaca acgagggcag gcggagcaag agaggcgcca gaaggctgaa gcggcggagg
cggcatagaa tccagagagt gaagaagctg ctgttcgact acaacctgct gaccgaccac
agcgagctga gcggcatcaa cccctacgag gccagagtga agggcctgag ccagaagctg
agcgaggaag agttctctgc cgccctgctg cacctggcca agagaagagg cgtgcacaac
gtgaacgagg tggaagagga caccggcaac gagctgtcca ccaaagagca gatcagccgg
aacagcaagg ccctggaaga gaaatacgtg gccgaactgc agctggaacg gctgaagaaa
gacggcgaag tgcggggcag catcaacaga ttcaagacca gcgactacgt gaaagaagcc
aaacagctgc tgaaggtgca gaaggcctac caccagctgg accagagctt catcgacacc
tacatcgacc tgctggaaac ccggcggacc tactatgagg gacctggcga gggcagcccc
ttcggctgga aggacatcaa agaatggtac gagatgctga tgggccactg cacctacttc
cccgaggaac tgcggagcgt gaagtacgcc tacaacgccg acctgtacaa cgccctgaac
gacctgaaca atctcgtgat caccagggac gagaacgaga agctggaata ttacgagaag
ttccagatca tcgagaacgt gttcaagcag aagaagaagc ccaccctgaa gcagatcgcc
aaagaaatcc tcgtgaacga agaggatatt aagggctaca gagtgaccag caccggcaag
cccgagttca ccaacctgaa ggtgtaccac gacatcaagg acattaccgc ccggaaagag
attattgaga acgccgagct gctggatcag attgccaaga tcctgaccat ctaccagagc
agcgaggaca tccaggaaga actgaccaat ctgaactccg agctgaccca ggaagagatc
gagcagatct ctaatctgaa gggctatacc ggcacccaca acctgagcct gaaggccatc
aacctgatcc tggacgagct gtggcacacc aacgacaacc agatcgctat cttcaaccgg
ctgaagctgg tgcccaagaa ggtggacctg tcccagcaga aagagatccc caccaccctg
gtggacgact tcatcctgag ccccgtcgtg aagagaagct tcatccagag catcaaagtg
atcaacgcca tcatcaagaa gtacggcctg cccaacgaca tcattatcga gctggcccgc
gagaagaact ccaaggacgc ccagaaaatg atcaacgaga tgcagaagcg gaaccggcag
accaacgagc ggatcgagga aatcatccgg accaccggca aagagaacgc caagtacctg
atcgagaaga tcaagctgca cgacatgcag gaaggcaagt gcctgtacag cctggaagcc
atccctctgg aagatctgct gaacaacccc ttcaactatg aggtggacca catcatcccc
agaagcgtgt ccttcgacaa cagcttcaac aacaaggtgc tcgtgaagca ggaagaaaac
agcaagaagg gcaaccggac cccattccag tacctgagca gcagcgacag caagatcagc
tacgaaacct tcaagaagca catcctgaat ctggccaagg gcaagggcag aatcagcaag
accaagaaag agtatctgct ggaagaacgg gacatcaaca ggttctccgt gcagaaagac
ttcatcaacc ggaacctggt ggataccaga tacgccacca gaggcctgat gaacctgctg
 
cggagctact tcagagtgaa caacctggac gtgaaagtga agtccatcaa tggcggcttc
accagctttc tgcggcggaa gtggaagttt aagaaagagc ggaacaaggg gtacaagcac
cacgccgagg acgccctgat cattgccaac gccgatttca tcttcaaaga gtggaagaaa
ctggacaagg ccaaaaaagt gatggaaaac cagatgttcg aggaaaagca ggccgagagc
atgcccgaga tcgaaaccga gcaggagtac aaagagatct tcatcacccc ccaccagatc
aagcacatta aggacttcaa ggactacaag tacagccacc gggtggacaa gaagcctaat
agagagctga ttaacgacac cctgtactcc acccggaagg acgacaaggg caacaccctg
atcgtgaaca atctgaacgg cctgtacgac aaggacaatg acaagctgaa aaagctgatc
aacaagagcc ccgaaaagct gctgatgtac caccacgacc cccagaccta ccagaaactg
aagctgatta tggaacagta cggcgacgag aagaatcccc tgtacaagta ctacgaggaa
accgggaact acctgaccaa gtactccaaa aaggacaacg gccccgtgat caagaagatt
aagtattacg gcaacaaact gaacgcccat ctggacatca ccgacgacta ccccaacagc
agaaacaagg tcgtgaagct gtccctgaag ccctacagat tcgacgtgta cctggacaat
ggcgtgtaca agttcgtgac cgtgaagaat ctggatgtga tcaaaaaaga aaactactac
gaagtgaata gcaagtgcta tgaggaagct aagaagctga agaagatcag caaccaggcc
gagtttatcg cctccttcta caacaacgat ctgatcaaga tcaacggcga gctgtataga
gtgatcggcg tgaacaacga cctgctgaac cggatcgaag tgaacatgat cgacatcacc
taccgcgagt acctggaaaa catgaacgac aagaggcccc ccaggatcat taagacaatc
gcctccaaga cccagagcat taagaagtac agcacagaca ttctgggcaa cctgtatgaa
gtgaaatcta agaagcaccc tcagatcatc aaaaagggc [SEQ ID NO: 29]
SEQ ID NO: 30 is set forth below. 
atgaagcgca actacatcct cggactggac atcggcatta cctccgtggg atacggcatc
atcgattacg aaactaggga tgtgatcgac gctggagtca ggctgttcaa agaggcgaac
gtggagaaca acgaggggcg gcgctcaaag aggggggccc gccggctgaa gcgccgccgc
agacatagaa tccagcgcgt gaagaagctg ctgttcgact acaaccttct gaccgaccac
tccgaacttt ccggcatcaa cccatatgag gctagagtga agggattgtc ccaaaagctg
tccgaggaag agttctccgc cgcgttgctc cacctcgcca agcgcagggg agtgcacaat
gtgaacgaag tggaagaaga taccggaaac gagctgtcca ccaaggagca gatcagccgg
aactccaagg ccctggaaga gaaatacgtg gcggaactgc aactggagcg gctgaagaaa
gacggagaag tgcgcggctc gatcaaccgc ttcaagacct cggactacgt gaaggaggcc
aagcagctcc tgaaagtgca aaaggcctat caccaacttg accagtcctt tatcgatacc
tacatcgatc tgctcgagac tcggcggact tactacgagg gtccagggga gggctcccca
tttggttgga aggatattaa ggagtggtac gaaatgctga tgggacactg cacatacttc
cctgaggagc tgcggagcgt gaaatacgca tacaacgcag acctgtacaa cgcgctgaac
gacctgaaca atctcgtgat cacccgggac gagaacgaaa agctcgagta ttacgaaaag
ttccagatta ttgagaacgt gttcaaacag aagaagaagc cgacactgaa gcagattgcc
aaggaaatcc tcgtgaacga agaggacatc aagggctatc gagtgacctc aacgggaaag
ccggagttca ccaatctgaa ggtctaccac gacatcaaag acattaccgc ccggaaggag
atcattgaga acgcggagct gttggaccag attgcgaaga ttctgaccat ctaccaatcc
tccgaggata ttcaggaaga actcaccaac ctcaacagcg aactgaccca ggaggagata
gagcaaatct ccaacctgaa gggctacacc ggaactcata acctgagcct gaaggccatc
aacttgatcc tggacgagct gtggcacacc aacgataacc agatcgctat tttcaatcgg
ctgaagctgg tccccaagaa agtggacctc tcacaacaaa aggagatccc tactaccctt
gtggacgatt tcattctgtc ccccgtggtc aagagaagct tcatacagtc aatcaaagtg
atcaatgcca ttatcaagaa atacggtctg cccaacgaca ttatcattga gctcgcccgc
gagaagaact cgaaggacgc ccagaagatg attaacgaaa tgcagaagag gaaccgacag
actaacgaac ggatcgaaga aatcatccgg accaccggga aggaaaacgc gaagtacctg
atcgaaaaga tcaagctcca tgacatgcag gaaggaaagt gtctgtactc gctggaggcc
attccgctgg aggacttgct gaacaaccct tttaactacg aagtggatca tatcattccg
aggagcgtgt cattcgacaa ttccttcaac aacaaggtcc tcgtgaagca ggaggaaaac
tcgaagaagg gaaaccgcac gccgttccag tacctgagca gcagcgactc caagatttcc
tacgaaacct tcaagaagca catcctcaac ctggcaaagg ggaagggtcg catctccaag
accaagaagg aatatctgct ggaagaaaga gacatcaaca gattctccgt gcaaaaggac
 
ttcatcaacc gcaacctcgt ggatactaga tacgctactc ggggtctgat gaacctcctg
agaagctact ttagagtgaa caatctggac gtgaaggtca agtcgattaa cggaggtttc
acctccttcc tgcggcgcaa gtggaagttc aagaaggaac ggaacaaggg ctacaagcac
cacgccgagg acgccctgat cattgccaac gccgacttca tcttcaaaga atggaagaaa
cttgacaagg ctaagaaggt catggaaaac cagatgttcg aagaaaagca ggccgagtct
atgcctgaaa tcgagactga acaggagtac aaggaaatct ttattacgcc acaccagatc
aaacacatca aggatttcaa ggattacaag tactcacatc gcgtggacaa aaagccgaac
agggaactga tcaacgacac cctctactcc acccggaagg atgacaaagg gaataccctc
atcgtcaaca accttaacgg cctgtacgac aaggacaacg ataagctgaa gaagctcatt
aacaagtcgc ccgaaaagtt gctgatgtac caccacgacc ctcagactta ccagaagctc
aagctgatca tggagcagta tggggacgag aaaaacccgt tgtacaagta ctacgaagaa
actgggaatt atctgactaa gtactccaag aaagataacg gccccgtgat taagaagatt
aagtactacg gcaacaagct gaacgcccat ctggacatca ccgatgacta ccctaattcc
cgcaacaagg tcgtcaagct gagcctcaag ccctaccggt ttgatgtgta ccttgacaat
ggagtgtaca agttcgtgac tgtgaagaac cttgacgtga tcaagaagga gaactactac
gaagtcaact ccaagtgcta cgaggaagca aagaagttga agaagatctc gaaccaggcc
gagttcattg cctccttcta taacaacgac ctgattaaga tcaacggcga actgtaccgc
gtcattggcg tgaacaacga tctcctgaac cgcatcgaag tgaacatgat cgacatcact
taccgggaat acctggagaa tatgaacgac aagcgcccgc cccggatcat taagactatc
gcctcaaaga cccagtcgat caagaagtac agcaccgaca tcctgggcaa cctgtacgag
gtcaaatcga agaagcaccc ccagatcatc aagaaggga [SEQ ID NO: 30]
SEQ ID NO: 31 is set forth below. 
ATGGCCCCAAAGAAGAAGCGGAAGGTCGGTATCCACGGAGTCCCAGCAGCCAAGCGGAACTACATCCTGGG
CCTGGACATCGGCATCACCAGCGTGGGCTACGGCATCATCGACTACGAGACACGGGACGTGATCGATGCCG
GCGTGCGGCTGTTCAAAGAGGCCAACGTGGAAAACAACGAGGGCAGGCGGAGCAAGAGAGGCGCCAGAAGG
CTGAAGCGGCGGAGGCGGCATAGAATCCAGAGAGTGAAGAAGCTGCTGTTCGACTACAACCTGCTGACCGA
CCACAGCGAGCTGAGCGGCATCAACCCCTACGAGGCCAGAGTGAAGGGCCTGAGCCAGAAGCTGAGCGAGG
AAGAGTTCTCTGCCGCCCTGCTGCACCTGGCCAAGAGAAGAGGCGTGCACAACGTGAACGAGGTGGAAGAG
GACACCGGCAACGAGCTGTCCACCAGAGAGCAGATCAGCCGGAACAGCAAGGCCCTGGAAGAGAAATACGT
GGCCGAACTGCAGCTGGAACGGCTGAAGAAAGACGGCGAAGTGCGGGGCAGCATCAACAGATTCAAGACCA
GCGACTACGTGAAAGAAGCCAAACAGCTGCTGAAGGTGCAGAAGGCCTACCACCAGCTGGACCAGAGCTTC
ATCGACACCTACATCGACCTGCTGGAAACCCGGCGGACCTACTATGAGGGACCTGGCGAGGGCAGCCCCTT
CGGCTGGAAGGACATCAAAGAATGGTACGAGATGCTGATGGGCCACTGCACCTACTTCCCCGAGGAACTGC
GGAGCGTGAAGTACGCCTACAACGCCGACCTGTACAACGCCCTGAACGACCTGAACAATCTCGTGATCACC
AGGGACGAGAACGAGAAGCTGGAATATTACGAGAAGTTCCAGATCATCGAGAACGTGTTCAAGCAGAAGAA
GAAGCCCACCCTGAAGCAGATCGCCAAAGAAATCCTCGTGAACGAAGAGGATATTAAGGGCTACAGAGTGA
CCAGCACCGGCAAGCCCGAGTTCACCAACCTGAAGGTGTACCACGACATCAAGGACATTACCGCCCGGAAA
GAGATTATTGAGAACGCCGAGCTGCTGGATCAGATTGCCAAGATCCTGACCATCTACCAGAGCAGCGAGGA
CATCCAGGAAGAACTGACCAATCTGAACTCCGAGCTGACCCAGGAAGAGATCGAGCAGATCTCTAATCTGA
AGGGCTATACCGGCACCCACAACCTGAGCCTGAAGGCCATCAACCTGATCCTGGACGAGCTGTGGCACACC
AACGACAACCAGATCGCTATCTTCAACCGGCTGAAGCTGGTGCCCAAGAAGGTGGACCTGTCCCAGCAGAA
AGAGATCCCCACCACCCTGGTGGACGACTTCATCCTGAGCCCCGTCGTGAAGAGAAGCTTCATCCAGAGCA
TCAAAGTGATCAACGCCATCATCAAGAAGTACGGCCTGCCCAACGACATCATTATCGAGCTGGCCCGCGAG
AAGAACTCCAAGGACGCCCAGAAAATGATCAACGAGATGCAGAAGCGGAACCGGCAGACCAACGAGCGGAT
CGAGGAAATCATCCGGACCACCGGCAAAGAGAACGCCAAGTACCTGATCGAGAAGATCAAGCTGCACGACA
TGCAGGAAGGCAAGTGCCTGTACAGCCTGGAAGCCATCCCTCTGGAAGATCTGCTGAACAACCCCTTCAAC
TATGAGGTGGACCACATCATCCCCAGAAGCGTGTCCTTCGACAACAGCTTCAACAACAAGGTGCTCGTGAA
GCAGGAAGAAAACAGCAAGAAGGGCAACCGGACCCCATTCCAGTACCTGAGCAGCAGCGACAGCAAGATCA
GCTACGAAACCTTCAAGAAGCACATCCTGAATCTGGCCAAGGGCAAGGGCAGAATCAGCAAGACCAAGAAA
GAGTATCTGCTGGAAGAACGGGACATCAACAGGTTCTCCGTGCAGAAAGACTTCATCAACCGGAACCTGGT
GGATACCAGATACGCCACCAGAGGCCTGATGAACCTGCTGCGGAGCTACTTCAGAGTGAACAACCTGGACG
TGAAAGTGAAGTCCATCAATGGCGGCTTCACCAGCTTTCTGCGGCGGAAGTGGAAGTTTAAGAAAGAGCGG
AACAAGGGGTACAAGCACCACGCCGAGGACGCCCTGATCATTGCCAACGCCGATTTCATCTTCAAAGAGTG
 
GAAGAAACTGGACAAGGCCAAAAAAGTGATGGAAAACCAGATGTTCGAGGAAAGGCAGGCCGAGAGCATGC
CCGAGATCGAAACCGAGCAGGAGTACAAAGAGATCTTCATCACCCCCCACCAGATCAAGCACATTAAGGAC
TTCAAGGACTACAAGTACAGCCACCGGGTGGACAAGAAGCCTAATAGAGAGCTGATTAACGACACCCTGTA
CTCCACCCGGAAGGACGACAAGGGCAACACCCTGATCGTGAACAATCTGAACGGCCTGTACGACAAGGACA
ATGACAAGCTGAAAAAGCTGATCAACAAGAGCCCCGAAAAGCTGCTGATGTACCACCACGACCCCCAGACC
TACCAGAAACTGAAGCTGATTATGGAACAGTACGGCGACGAGAAGAATCCCCTGTACAAGTACTACGAGGA
AACCGGGAACTACCTGACCAAGTACTCCAAAAAGGACAACGGCCCCGTGATCAAGAAGATTAAGTATTACG
GCAACAAACTGAACGCCCATCTGGACATCACCGACGACTACCCCAACAGCAGAAACAAGGTCGTGAAGCTG
TCCCTGAAGCCCTACAGATTCGACGTGTACCTGGACAATGGCGTGTACAAGTTCGTGACCGTGAAGAATCT
GGATGTGATCAAAAAAGAAAACTACTACGAAGTGAATAGCAAGTGCTATGAGGAAGCTAAGAAGCTGAAGA
AGATCAGCAACCAGGCCGAGTTTATCGCCTCCTTCTACAACAACGATCTGATCAAGATCAACGGCGAGCTG
TATAGAGTGATCGGCGTGAACAACGACCTGCTGAACCGGATCGAAGTGAACATGATCGACATCACCTACCG
CGAGTACCTGGAAAACATGAACGACAAGAGGCCCCCCAGGATCATTAAGACAATCGCCTCCAAGACCCAGA
GCATTAAGAAGTACAGCACAGACATTCTGGGCAACCTGTATGAAGTGAAATCTAAGAAGCACCCTCAGATC
ATCAAAAAGGGCAAAAGGCCGGCGGCCACGAAAAAGGCCGGCCAGGCAAAAAAGAAAAAG [SEQ ID
NO: 31]
SEQ ID NO: 32 is set forth below.

ACCGGTGCCA CCATGTACCC ATACGATGTT CCAGATTACG CTTCGCCGAA GAAAAAGCGC
AAGGTCGAAG CGTCCATGAA AAGGAACTAC ATTCTGGGGC TGGACATCGG GATTACAAGC
GTGGGGTATG GGATTATTGA CTATGAAACA AGGGACGTGA TCGACGCAGG CGTCAGACTG
TTCAAGGAGG CCAACGTGGA AAACAATGAG GGACGGAGAA GCAAGAGGGG AGCCAGGCGC
CTGAAACGAC GGAGAAGGCA CAGAATCCAG AGGGTGAAGA AACTGCTGTT CGATTACAAC
CTGCTGACCG ACCATTCTGA GCTGAGTGGA ATTAATCCTT ATGAAGCCAG GGTGAAAGGC
CTGAGTCAGA AGCTGTCAGA GGAAGAGTTT TCCGCAGCTC TGCTGCACCT GGCTAAGCGC
CGAGGAGTGC ATAACGTCAA TGAGGTGGAA GAGGACACCG GCAACGAGCT GTCTACAAAG
GAACAGATCT CACGCAATAG CAAAGCTCTG GAAGAGAAGT ATGTCGCAGA GCTGCAGCTG
GAACGGCTGA AGAAAGATGG CGAGGTGAGA GGGTCAATTA ATAGGTTCAA GACAAGCGAC
TACGTCAAAG AAGCCAAGCA GCTGCTGAAA GTGCAGAAGG CTTACCACCA GCTGGATCAG
AGCTTCATCG ATACTTATAT CGACCTGCTG GAGACTCGGA GAACCTACTA TGAGGGACCA
GGAGAAGGGA GCCCCTTCGG ATGGAAAGAC ATCAAGGAAT GGTACGAGAT GCTGATGGGA
CATTGCACCT ATTTTCCAGA AGAGCTGAGA AGCGTCAAGT ACGCTTATAA CGCAGATCT
TACAACGCCC TGAATGACCT GAACAACCTG GTCATCACCA GGGATGAAAA CGAGAAACTG
GAATACTATG AGAAGTTCCA GATCATCGAA AACGTGTTTA AGCAGAAGAA AAAGCCTACA
CTGAAACAGA TTGCTAAGGA GATCCTGGTC AACGAAGAGG ACATCAAGGG CTACCGGGTG
ACAAGCACTG GAAAACCAGA GTTCACCAAT CTGAAAGTGT ATCACGATAT TAAGGACATC
ACAGCACGGA AAGAAATCAT TGAGAACGCC GAACTGCTGG ATCAGATTGC TAAGATCCTG
ACTATCTACC AGAGCTCCGA GGACATCCAG GAAGAGCTGA CTAACCTGAA CAGCGAGCTG
ACCCAGGAAG AGATCGAACA GATTAGTAAT CTGAAGGGGT ACACCGGAAC ACACAACCTG
TCCCTGAAAG CTATCAATCT GATTCTGGAT GAGCTGTGGC ATACAAACGA CAATCAGATT
GCAATCTTTA ACCGGCTGAA GCTGGTCCCA AAAAAGGTGG ACCTGAGTCA GCAGAAAGAG
ATCCCAACCA CACTGGTGGA CGATTTCATT CTGTCACCCG TGGTCAAGCG GAGCTTCATC
CAGAGCATCA AAGTGATCAA CGCCATCATC AAGAAGTACG GCCTGCCCAA TGATATCATT
ATCGAGCTGG CTAGGGAGAA GAACAGCAAG GACGCACAGA AGATGATCAA TGAGATGCAG
AAACGAAACC GGCAGACCAA TGAACGCATT GAAGAGATTA TCCGAACTAC CGGGAAAGAG
AACGCAAAGT ACCTGATTGA AAAAATCAAG CTGCACGATA TGCAGGAGGG AAAGTGTCTG
TATTCTCTGG AGGCCATCCC CCTGGAGGAC CTGCTGAACA ATCCATTCAA CTACGAGGTC
GATCATATTA TCCCCAGAAG CGTGTCCTTC GACAATTCCT TTAACAACAA GGTGCTGGTC
AAGCAGGAAG AGAACTCTAA AAAGGGCAAT AGGACTCCTT TCCAGTACCT GTCTAGTTCA
GATTCCAAGA TCTCTTACGA AACCTTTAAA AAGCACATTC TGAATCTGGC CAAAGGAAAG
GGCCGCATCA GCAAGACCAA AAAGGAGTAC CTGCTGGAAG AGCGGGACAT CAACAGATTC
TCCGTCCAGA AGGATTTTAT TAACCGGAAT CTGGTGGACA CAAGATACGC TACTCGCGGC
CTGATGAATC TGCTGCGATC CTATTTCCGG GTGAACAATC TGGATGTGAA AGTCAAGTCC
ATCAACGGCG GGTTCACATC TTTTCTGAGG CGCAAATGGA AGTTTAAAAA GGAGCGCAAC

AAAGGGTACA AGCACCATGC CGAAGATGCT CTGATTATCG CAAATGCCGA CTTCATCTTT
AAGGAGTGGA AAAAGCTGGA CAAAGCCAAG AAAGTGATGG AGAACCAGAT GTTCGAAGAG
AAGCAGGCCG AATCTATGCC CGAAATCGAG ACAGAACAGG AGTACAAGGA GATTTTCATC
ACTCCTCACC AGATCAAGCA TATCAAGGAT TTCAAGGACT ACAAGTACTC TCACCGGGTG
GATAAAAAGC CCAACAGAGA GCTGATCAAT GACACCCTGT ATAGTACAAG AAAAGACGAT
AAGGGGAATA CCCTGATTGT GAACAATCTG AACGGACTGT ACGACAAAGA TAATGACAAG
CTGAAAAAGC TGATCAACAA AAGTCCCGAG AAGCTGCTGA TGTACCACCA TGATCCTCAG
ACATATCAGA AACTGAAGCT GATTATGGAG CAGTACGGCG ACGAGAAGAA CCCACTGTAT
AAGTACTATG AAGAGACTGG GAACTACCTG ACCAAGTATA GCAAAAAGGA TAATGGCCCC
GTGATCAAGA AGATCAAGTA CTATGGGAAC AAGCTGAATG CCCATCTGGA CATCACAGAC
GATTACCCTA ACAGTCGCAA CAAGGTGGTC AAGCTGTCAC TGAAGCCATA CAGATTCGAT
GTCTATCTGG ACAACGGCGT GTATAAATTT GTGACTGTCA AGAATCTGGA TGTCATCAAA
AAGGAGAACT ACTATGAAGT GAATAGCAAG TGCTACGAAG AGGCTAAAAA GCTGAAAAAG
ATTAGCAACC AGGCAGAGTT CATCGCCTCC TTTTACAACA ACGACCTGAT TAAGATCAAT
GGCGAACTGT ATAGGGTCAT CGGGGTGAAC AATGATCTGC TGAACCGCAT TGAAGTGAAT
ATGATTGACA TCACTTACCG AGAGTATCTG GAAAACATGA ATGATAAGCG CCCCCCTCGA
ATTATCAAAA CAATTGCCTC TAAGACTCAG AGTATCAAAA AGTACTCAAC CGACATTCTG
GGAAACCTGT ATGAGGTGAA GAGCAAAAAG CACCCTCAGA TTATCAAAAA GGGCTAAGAA
TTC [SEQ ID NO: 32]

An amino acid sequence of an *S. aureus* Cas9 molecule is set forth in SEQ ID NO: 33, which is provided below.

MKRNYILGLDIGITSVGYGIIDYETRDVIDAGVRLFKEANVENNEGRRSKRGARRLKRRR
RHRIQRVKKLLFDYNLLTDHSELSGINPYEARVKGLSQKLSEEEFSAALLHLAKRRGVHN
VNEVEEDTGNELSTKEQISRNSKALEEKYVAELQLERLKKDGEVRGSINRFKTSDYVKEA
KQLLKVQKAYHQLDQSFIDTYIDLLETRRTYYEGPGEGSPFGWKDIKEWYEMLMGHCTYF
PEELRSVKYAYNADLYNALNDLNNLVITRDENEKLEYYEKFQIIENVFKQKKKPTLKQIA
KEILVNEEDIKGYRVTSTGKPEFTNLKVYHDIKDITARKEIIENAELLDQIAKILTIYQS
SEDIQEELTNLNSELTQEEIEQISNLKGYTGTHNLSLKAINLILDELWHTNDNQIAIFNR
LKLVPKKVDLSQQKEIPTTLVDDFILSPVVKRSFIQSIKVINAIIKKYGLPNDIIIELAR
EKNSKDAQKMINEMQKRNRQTNERIEEIIRTTGKENAKYLIEKIKLHDMQEGKCLYSLEA
IPLEDLLNNPFNYEVDHIIPRSVSFDNSFNNKVLVKQEENSKKGNRTPFQYLSSSDSKIS
YETFKKHILNLAKGKGRISKTKKEYLLEERDINRFSVQKDFINRNLVDTRYATRGLMNLL
RSYFRVNNLDVKVKSINGGFTSFLRRKWKFKKERNKGYKHHAEDALIIANADFIFKEWKK
LDKAKKVMENQMFEEKQAESMPEIETEQEYKEIFITPHQIKHIKDFKDYKYSHRVDKKPN
RELINDTLYSTRKDDKGNTLIVNNLNGLYDKDNDKLKKLINKSPEKLLMYHHDPQTYQKL
KLIMEQYGDEKNPLYKYYEETGNYLTKYSKKDNGPVIKKIKYYGNKLNAHLDITDDYPNS
RNKVVKLSLKPYRFDVYLDNGVYKFVTVKNLDVIKKENYYEVNSKCYEEAKKLKKISNQA
EFIASFYNNDLIKINGELYRVIGVNNDLLNRIEVNMIDITYREYLENMNDKRPPRIIKTI
ASKTQSIKKYSTDILGNLYEVKSKKHPQIIKKG [SEQ ID NO: 33]

### 2.2.2. gRNA molecules

The CRISPR/Cas9 system includes at least two gRNA molecules. gRNA molecules provide the targeting of the CRISPR/Cas9-based system. gRNA is a fusion of two noncoding RNAs: a crRNA and a tracrRNA. gRNA can target any desired DNA sequence by exchanging the sequence encoding a 20 bp protospacer which confers targeting specificity through complementary base pairing with the desired DNA target. gRNA mimics the naturally occurring crRNA:tracrRNA duplex involved in the Type II Effector system. This duplex, which can include, for example, a 42-nucleotide crRNA and a 75 -nucleotide tracrRNA, acts as a guide for the Cas9 to cleave the target nucleic acid. The "target region", "target sequence" or "protospacer" as used interchangeably herein refers to the region of the target gene (e.g., a *dystrophin* gene) to which the CRISPR/Cas9-based system targets. The CRISPR/Cas9-based system can include two or more gRNA molecules, which target different DNA sequences. The target DNA sequences can be overlapping. The target sequence or protospacer is followed by a PAM sequence at the 3' end of the protospacer. Different Type II systems have differing PAM requirements.

The number of gRNA molecule encoded by a presently disclosed genentic construct (e.g., an AAV vector) can be at least 2 different gRNAs, at least 3 different gRNAs at least 4 different gRNAs, at least 5 different gRNAs, at least 6 different gRNAs, at least 7 different gRNAs, at least 8 different gRNAs, at least 9 different gRNAs, at least 10 different gRNAs, at least 11 different gRNAs, at least 12 different gRNAs, at least 13 different gRNAs, at least 14 different gRNAs, at least 15 different gRNAs, at least 16 different gRNAs, at least 17 different gRNAs, at least 18 different gRNAs, at least 18 different gRNAs, at least 20 different gRNAs, at least 25 different gRNAs, at least 30 different gRNAs, at least 35 different gRNAs, at least 40 different gRNAs, at least 45 different gRNAs, or at least 50 different gRNAs. In the present disclosure, the genetic construct (e.g., an AAV vector) encodes two gRNA molecules, *i.e.*, a first gRNA molecule, and a second gRNA molecule.

gRNA molecule comprises a targeting domain, which is a complementary polynucleotide sequence of the target DNA sequence followed by a PAM sequence. gRNA molecule can comprise a "G" at the 5' end of the targeting domain. The targeting domains of the gRNA molecules are 21 nucleotides in length.

gRNA can target at least one of exons, introns, the promoter region, the enhancer region, the transcribed region of the *dystrophin* gene. In certain embodiments, the gRNA molecule targets intron 51 of the human *dystrophin* gene. In certain embodiments, the gRNA molecule targets exon 51 of the human *dystrophin* gene.

### 2.2.3. Altering a Dystrophin Gene

The presently disclosed vector encodes gRNA molecules that targets a *dystrophin* gene (e.g., human *dystrophin* gene). The gRNA molecules can bind and recognize a target region. The target regions can be chosen immediately upstream of possible out-of- frame stop codons such that insertions or deletions during the repair process restore the dystrophin reading frame by frame conversion. Target regions can also be splice acceptor sites or splice donor sites, such that insertions or deletions during the repair process disrupt splicing and restore the dystrophin reading frame by splice site disruption and exon exclusion. Target regions can also be aberrant stop codons such that insertions or deletions during the repair process restore the dystrophin reading frame by eliminating or disrupting the stop codon.

Single or multiplexed gRNAs can be designed to restore the dystrophin reading frame by targeting the mutational hotspot at exon 51 or and introducing either intraexonic small insertions and deletions, or excision of exon 51. Following treatment with a presently disclosed vector, dystrophin expression can be restored in Duchenne patient muscle cells *in vitro.* Human dystrophin was detected *in vivo* following transplantation of genetically corrected patient cells into immunodeficient mice. Significantly, the unique multiplex gene editing capabilities of the CRISPR/Cas9 system enable efficiently generating large deletions of this mutational hotspot region that can correct up to 62% of patient mutations by universal or patient- specific gene editing approaches.

The presently disclosed vectors can generate deletions in the *dystrophin* gene, e.g., the human *dystrophin* gene. In certain embodiments, the vector is configured to form two double stand breaks (a first double strand break and a second double strand break) in two introns (a first intron and a second intron) flanking a target position of the *dystrophin* gene, thereby deleting a segment of the *dystrophin* gene comprising the dystrophin target position. A "dystrophin target position" can be a dystrophin exonic target position or a dystrophin intra-exonic target position, as described herein. Deletion of the *dystrophin* exonic target position can optimize the *dystrophin* sequence of a subject suffering from Duchenne muscular dystrophy, e.g., it can increase the function or activity of the encoded dystrophin protein, or results in an improvement in the disease state of the subject. In certain embodiments, excision of the *dystrophin* exonic target position restores reading frame. The *dystrophin* exonic target position can comprise one or more exons of the *dystrophin* gene. In certain embodiments, the dystrophin target position comprises exon 51 of the *dystrophin* gene (e.g., human *dystrophin* gene).

In certain embodiments, Duchenne Muscular Dystrophy (DMD) refers to a recessive, fatal, X-linked disorder that results in muscle degeneration and eventual death. DMD is a common hereditary monogenic disease and occurs in 1 in 3500 males. DMD is the result of inherited or spontaneous mutations that cause nonsense or frame shift mutations in the dystrophin gene. The majority of dystrophin mutations that cause DMD are deletions of exons that disrupt the reading frame and cause premature translation termination in the dystrophin gene. DMD patients typically lose the ability to physically support themselves during childhood, become progressively weaker during the teenage years, and die in their twenties.

The presently disclosed vector can mediate highly efficient gene editing at exon 51 of a *dystrophin* gene (e.g., the human *dystrophin* gene). The presently disclosed vector restores dystrophin protein expression in cells from DMD patients.

Exon 51 is frequently adjacent to frame-disrupting deletions in DMD. Elimination of exon 51 from the *dystrophin* transcript by exon skipping can be used to treat approximately 15% of all DMD patients. This class of *dystrophin* mutations is ideally suited for permanent correction by NHEJ-based genome editing and HDR. The genetic constructs (e.g., vectors) described herein have been developed for targeted modification of exon 51 in the human *dystrophin* gene. The presently disclosed vector can be transfected into human DMD cells and mediates efficient gene modification and conversion to the correct reading frame. Protein restoration is concomitant with frame restoration and detected in a bulk population of CRISPR/Cas9-based system-treated cells.

In the present disclosure, the vector encodes a first gRNA molecule and a second gRNA molecule, and at least one *S.aureus* Cas9 molecule that recognizes a PAM of either NNGRRT (SEQ ID NO:24) or NNGRRV (SEQ ID NO:25). In some embodiments the vector is configured to form a first and a second double strand break in a first and a second intron flanking exon 51 of the human *dystrophin* gene, respectively, thereby deleting a segment of the *dystrophin* gene comprising exon 51.

The deletion efficiency of the presently disclosed vectors can be related to the deletion size, *i.e.*, the size of the segment deleted by the vectors. In certain embodiments, the length or size of specific deletions is determined by the distance between the PAM sequences in the gene being targeted (e.g., a *dystrophin* gene). In certain embodiments, a specific deletion of a segment of the *dystrophin* gene, which is defined in terms of its length and a sequence it comprises (e.g., exon 51), is the result of breaks made adjacent to specific PAM sequences within the target gene (e.g., a *dystrophin* gene).

In certain embodiments, the deletion size is about 800-72,000 base pairs (bp), e.g., about 800-900, about 900-1000, about 1200-1400, about 1500-2600, about 2600-2700, about 3000-3300, about 5200-5500, about 20,000-30,000, about 35,000-45,000, or about 60,000-72,000. In certain embodiments, the deletion size is about 800-900, about 1500-2600, about 5200-5500, about 20,000-30,000, about 35,000-45,000, or about 60,000-72,000 bp. In certain embodiments, the deletion size is 806 base pairs, 867 base pairs, 1,557 base pairs, 2,527 base pairs, 5,305 base pairs, 5,415 base pairs, 20,768 base pairs, 27,398 base pairs, 36,342 base pairs, 44,269 base pairs, 60,894 base pairs, or 71,832 base pairs. In certain embodiments, the deletion size is about 900-1000, about 1200-1400, about 1500-2600, about 2600-2700 bp, or about 3000-3300. In certain embodiments, the deletion size is selected from the group consisting of 972 bp, 1723 bp, 893 bp, 2665 bp, 1326 bp, 2077 bp, 1247 bp, 3019 bp, 1589 bp, 2340 bp, 1852 bp, and 3282 bp. In certain embodiments, the deletion size is larger than about 150 kilobase pairs (kb), e.g., about 300-400 kb. In certain embodiments, the deletion size is about 300-400 kb. In certain embodiments, the deletion size is 341 kb. In certain embodiments, the deletion size is about 100-150 kb. In certain embodiments, the deletion size is 146,500 bp.

In the present disclosure, the vector encodes a least one *S.aureus* Cas9 molecule and a pair of two gRNA molecules as shown in the first entry in Table 1.

**Table 1**

| **Guide Pair** | **gRNA No.** | **Targeting Domain Sequence** | **Length** | **Avg Del Effy (%)** | **PlaUe Normalized Avg Del Eff (a.u.)** | **Norm Stdev Del Eff** | **Deletion Size (bp)** |
|---|---|---|---|---|---|---|---|
| 84+68 | 84 | GUGUUAUUACUUGCUACUGCA (SEQ ID NO: 1) | 21 | 31.8 | 2.39 | 0.55 | 2527 |
| | 68 | GUGUAUUGCUUGUACUACUCA (SEQ ID NO: 2) | 21 | | | | |

| ENTRIES BELOW IN TABLE 1 ARE NOT IN ACCORDANCE WITH THE PRESENT INVENTION | | | | | | | |
|---|---|---|---|---|---|---|---|
| 82+68 | 82 | GUUUAAAUGUAAAUAGCUCAG (SEQ ID NO: 3) | 21 | 28.92 | 2.09 | 0.5 | 1557 |
| | 68 | GUGUAUUGCUUGUACUACUCA (SEQ ID NO: 2) | 21 | | | | |
| 1+9 | 1 | GAAUUUUCAAUGAUGUUCUGGG (SEQ ID NO: 4) | 22 | 27.87 | 2.04 | 0.31 | 5415 |
| | 9 | GAACUGGUGGGAAAUGGUCUAG (SEQ ID NO: 5) | 22 | | | | |
| 94+9 | 94 | GUUUCAUUGGCUUUGAUUUCCC (SEQ ID NO: 6) | 22 | 26.66 | 2.01 | 056 | 806 |
| | 9 | GAACUGGUGGGAAAUGGUCUAG (SEQ ID NO: 5) | 22 | | | | |
| 86+68 | 86 | GGCAAUUCUCCUGAAUAGAAA (SEQ ID NO: 7) | 21 | 27.8 | 2 | 0.38 | 5305 |
| | 68 | GUGUAUUGCUUGUACUACUCA (SEQ ID NO: 2) | 21 | | | | |
| 94+97 | 94 | GUUUCAUUGGCUUUGAUUUCCC (SEQ ID NO: 6) | 22 | 25.4 | 1.85 | 0.52 | 867 |
| | 97 | GAUUAUACUUAGGCUGAAUAGU (SEQ ID NO: 8) | 22 | | | | |
| 62+38 | 62 | GACUUCCAGAAUUAUGUGUUC (SEQ ID NO: 9) | 21 | 22.23 | 1.64 | 0.28 | 20768 |
| | 38 | GUGAGGGCCUGACACAUGGUA (SEQ ID NO: 10) | 21 | | | | |
| 55+20 | 55 | GUGAAGAUCAUUUCUUGGUAG (SEQ ID NO: 11) | 21 | 21.02 | 1.56 | 0.33 | 44269 |
| | 20 | GCACAGUCAGAACUAGUGUGC (SEQ ID NO: 12) | 21 | | | | |
| 59+38 | 59 | GAGUAAGCCCGAUCAUUAUUG (SEQ ID NO: 13) | 21 | 20.15 | 1.51 | 0.37 | 27398 |
| | 38 | GUGAGGGCCUGACACAUGGUA (SEQ ID NO: 10) | 21 | | | | |
| 54+31 | 54 | GGAAGGGACAUAUUCUAUGGG (SEQ ID NO: 14) | 21 | 19.83 | 1.43 | 048 | 71832 |
| | 31 | GACCACAAGCUGACUUGGGGG (SEQ ID NO: 15) | 21 | | | | |
| 55+38 | 55 | GUGAAGAUCAUUUCUUGGUAG (SEQ ID NO: 11) | 21 | 18.44 | 1.32 | 0.32 | 36342 |
| | 38 | GUGAGGGCCUGACACAUGGUA (SEQ ID NO: 10) | 21 | | | | |
| 54+26 | 54 | GGAAGGGACAUAUUCUAUGGG (SEQ ID NO: 14) | 21 | 13.37 | 0.95 | 0.11 | 60894 |
| | 26 | GGAUUUGUAUCCAUUAUCUGG (SEQ ID NO: 16) | 21 | | | | |

In certain embodiments, the vector is an AAV vector. In certain embodiments, the AAV vector is a modified AAV vector. The modified AAV vector can have enhanced cardiac and skeletal muscle tissue tropism. The modified AAV vector can deliver and express the CRISPR/Cas9 system described herein in the cell of a mammal. For example, the modified AAV vector can be an AAV-SASTG vector (Piacentino et al. (2012) Human Gene Therapy 23:635-646). The modified AAV vector can deliver the CRISPR/Cas9 system described herein to skeletal and cardiac muscle *in vivo.* The modified AAV vector can be based on one or more of several capsid types, including AAVl, AAV2, AAV5, AAV6, AAV8, and AAV9. The modified AAV vector can be based on AAV2 pseudotype with alternative muscle-tropic AAV capsids, such as AAV2/1, AAV2/6, AAV2/7, AAV2/8, AAV2/9, AAV2.5 and AAV/SASTG vectors that efficiently transduce skeletal muscle or cardiac muscle by systemic and local delivery (Seto et al. Current Gene Therapy (2012) 12: 139-151).

### 3. Compositions

The presently disclosed subject matter provides for compositions comprising the above-described genetic vectors. The compositions can be in a pharmaceutical composition. The pharmaceutical compositions can be formulated according to the mode of administration to be used. In cases where pharmaceutical compositions are injectable pharmaceutical compositions, they are sterile, pyrogen free and particulate free. An isotonic formulation is preferably used. Generally, additives for isotonicity may include sodium chloride, dextrose, mannitol, sorbitol and lactose. In certain embodiments, isotonic solutions such as phosphate buffered saline are preferred. Stabilizers include gelatin and albumin. In certain embodiments, a vasoconstriction agent is added to the formulation.

The composition may further comprise a pharmaceutically acceptable excipient. The pharmaceutically acceptable excipient may be functional molecules as vehicles, adjuvants, carriers, or diluents. The pharmaceutically acceptable excipient may be a transfection facilitating agent, which may include surface active agents, such as immune-stimulating complexes (ISCOMS), Freunds incomplete adjuvant, LPS analog including monophosphoryl lipid A, muramyl peptides, quinone analogs, vesicles such as squalene and squalene, hyaluronic acid, lipids, liposomes, calcium ions, viral proteins, polyanions, polycations, or nanoparticles, or other known transfection facilitating agents.

The transfection facilitating agent is a polyanion, polycation, including poly-L- glutamate (LGS), or lipid. The transfection facilitating agent is poly-L-glutamate, and more preferably, the poly-L-glutamate is present in the composition for genome editing in skeletal muscle or cardiac muscle at a concentration less than 6 mg/ml. The transfection facilitating agent may also include surface active agents such as immune-stimulating complexes (ISCOMS), Freunds incomplete adjuvant, LPS analog including monophosphoryl lipid A, muramyl peptides, quinone analogs and vesicles such as squalene and squalene, and hyaluronic acid may also be used administered in conjunction with the genetic construct. In certain embodiments, the DNA vector encoding the composition may also include a transfection facilitating agent such as lipids, liposomes, including lecithin liposomes or other liposomes known in the art, as a DNA-liposome mixture (see for example WO9324640), calcium ions, viral proteins, polyanions, polycations, or nanoparticles, or other known transfection facilitating agents. Preferably, the transfection facilitating agent is a polyanion, polycation, including poly-L-glutamate (LGS), or lipid. 17.

### 4. Correction of a Mutant Gene

The presently disclosed vector may be used to correct a mutant gene in a subject.

Correcting comprises changing a mutant gene that encodes a truncated protein or no protein at all, such that a full-length functional or partially full-length functional protein expression is obtained. Correcting a mutant gene can comprise replacing the region of the gene that has the mutation or replacing the entire mutant gene with a copy of the gene that does not have the mutation with a repair mechanism such as homology-directed repair (HDR). Correcting a mutant gene can also comprise repairing a frameshift mutation that causes a premature stop codon, an aberrant splice acceptor site or an aberrant splice donor site, by generating a double stranded break in the gene that is then repaired using non-homologous end joining (NHEJ). NHEJ can add or delete at least one base pair during repair which may restore the proper reading frame and eliminate the premature stop codon. Correcting a mutant gene can also comprise disrupting an aberrant splice acceptor site or splice donor sequence. Correcting can also comprise deleting a non-essential gene segment by the simultaneous action of two nucleases on the same DNA strand in order to restore the proper reading frame by removing the DNA between the two nuclease target sites and repairing the DNA break by NHEJ.

In certain embodiments, "Homology-directed repair" or "HDR" refers to a mechanism in cells to repair double strand DNA lesions when a homologous piece of DNA is present in the nucleus, mostly in G2 and S phase of the cell cycle. HDR uses a donor DNA template to guide repair and may be used to create specific sequence changes to the genome, including the targeted addition of whole genes. If a donor template is provided along with the CRISPR/Cas9-based systems, then the cellular machinery will repair the break by homologous recombination, which is enhanced several orders of magnitude in the presence of DNA cleavage. When the homologous DNA piece is absent, nonhomologous end joining may take place instead.

In certain embodiments, a donor DNA or a donor template refers to a double-stranded DNA fragment or molecule that includes at least a portion of the gene of interest, e.g., dystrophin gene. The donor DNA may encode a full-functional protein or a partially-functional protein.

In certain embodiments, "Non-homologous end joining (NHEJ) pathway" refers to a pathway that repairs double-strand breaks in DNA by directly ligating the break ends without the need for a homologous template. The template-independent re-ligation of DNA ends by NHEJ is a stochastic, error-prone repair process that introduces random micro-insertions and micro-deletions (indels) at the DNA breakpoint. This method may be used to intentionally disrupt, delete, or alter the reading frame of targeted gene sequences. NHEJ typically uses short homologous DNA sequences called microhomologies to guide repair. These microhomologies are often present in single-stranded overhangs on the end of double-strand breaks. When the overhangs are perfectly compatible, NHEJ usually repairs the break accurately, yet imprecise repair leading to loss of nucleotides may also occur, but is much more common when the overhangs are not compatible. In certain embodiments, NHEJ is a nuclease mediated NHEJ, which in certain embodiments, refers to NHEJ that is initiated a Cas9 molecule, cuts double stranded DNA. The presently disclosed vector or a composition comprising thereof may be administered to the skeletal muscle or cardiac muscle of the subject for genome editing in skeletal muscle or cardiac muscle. Genome editing comprises knocking out a gene, such as a mutant gene or a normal gene. Genome editing can be used to treat disease or enhance muscle repair by changing the gene of interest.

Use of the vectors or compositions comprising thereof to deliver the CRISPR/Cas9 system disclosed herein to the skeletal muscle or cardiac muscle can restore the expression of a full-functional or partially-functional protein with a repair template or donor DNA, which can replace the entire gene or the region containing the mutation. The CRISPR/Cas9 system can be used to introduce site-specific double strand breaks at targeted genomic loci. Site-specific double-strand breaks are created when the the CRISPR/Cas9 system binds to a target DNA sequences, thereby permitting cleavage of the target DNA. The CRISPR/Cas9-based system has the advantage of advanced genome editing due to their high rate of successful and efficient genetic modification. This DNA cleavage may stimulate the natural DNA-repair machinery, leading to one of two possible repair pathways: homology-directed repair (HDR) or the non-homologous end joining (NHE J) pathway.

The present disclosure relates to genome editing with a CRISPR/Cas9 system without a repair template, which can efficiently correct the reading frame and restore the expression of a functional protein involved in a genetic disease. The disclosed CRISPR/Cas9 system can involve using homology-directed repair or nuclease-mediated non-homologous end joining (NHEJ)-based correction approaches, which enable efficient correction in proliferation-limited primary cell lines that may not be amenable to homologous recombination or selection-based gene correction. This strategy integrates the rapid and robust assembly of active CRISPR/Cas9 systems with an efficient gene editing method for the treatment of genetic diseases caused by mutations in nonessential coding regions that cause frameshifts, premature stop codons, aberrant splice donor sites or aberrant splice acceptor sites.

Restoration of protein expression from an endogenous mutated gene may be through template-free NHEJ-mediated DNA repair. In contrast to a transient method targeting the target gene RNA, the correction of the target gene reading frame in the genome by a transiently expressed CRISPR/Cas9 system may lead to permanently restored target gene expression by each modified cell and all of its progeny.

Nuclease mediated NHEJ gene correction can correct the mutated target gene and offers several potential advantages over the HDR pathway. For example, NHEJ does not require a donor template, which may cause nonspecific insertional mutagenesis. In contrast to HDR, NHEJ operates efficiently in all stages of the cell cycle and therefore may be effectively exploited in both cycling and post-mitotic cells, such as muscle fibers. This provides a robust, permanent gene restoration alternative to oligonucleotide-based exon skipping or pharmacologic forced read-through of stop codons and could theoretically require as few as one drug treatment. NHEJ-based gene correction using a CRISPR/Cas9-based system may be combined with other existing *ex vivo* and *in vivo* platforms for cell- and gene -based therapies, in addition to the plasmid electroporation approach described here. For example, delivery of a CRISPR/Cas9-based system by mRNA-based gene transfer or as purified cell permeable proteins could enable a DNA- free genome editing approach that would circumvent any possibility of insertional mutagenesis.

Restoration of protein expression from an endogenous mutated gene may involve homology-directed repair. The present disclosure further includes administrating a donor template to the cell. The donor template can include a nucleotide sequence encoding a full-functional protein or a partially-functional protein. For example, the donor template can include a miniaturized dystrophin construct, termed minidystrophin ("minidys"), a full-functional dystrophin construct for restoring a mutant dystrophin gene, or a fragment of the dystrophin gene that after homology-directed repair leads to restoration of the mutant dystrophin gene.

The presently disclosed vector and composition may be used to correct a mutant *dystrophin* gene (e.g., a mutant human *dystrophin* gene) in a cell and treat a subject suffering from a genetic disease, such as DMD.

### 5. Use of the Presently Disclosed Vector to Treat a Disease -

The presently disclosed subject matter can be used to treat a subject in need thereof. The disclosed vector or composition may be administered to a tissue of a subject. The vector or composition may be administered to the skeletal muscle or cardiac muscle of the subject. The subject may be suffering from a skeletal muscle or cardiac muscle condition causing degeneration or weakness or a genetic disease. The subject may be suffering from Duchenne muscular dystrophy, as described above.

The disclosed vector or composition can be used for correcting the *dystrophin* gene and recovering full-functional or partially-functional protein expression of said mutated *dystrophin* gene. Use of the disclosed vector or composition may reduce the effects (e.g., clinical symptoms/indications) of DMD in a patient.

### 6. Delivery of Vector or Composition

Delivering of a vectoror a composition comprising thereof to a cell can be by the transfection or electroporation of the vector or compositions comprising thereof as a nucleic acid molecule that is expressed in the cell and delivered to the surface of the cell. The nucleic acid molecules can be electroporated using BioRad Gene Pulser Xcell or Amaxa Nucleofector lib devices. Several different buffers may be used, including BioRad electroporation solution, Sigma phosphate-buffered saline product #D8537 (PBS), Invitrogen OptiMEM I (OM), or Amaxa Nucleofector solution V (N.V.). Transfections can include a transfection reagent, such as Lipofectamine 2000.

Upon delivery to the tissue, and thereupon the vector into the cells of the mammal, the transfected cells will express the at least one Cas9 molecule and the two gRNA molecules. The genetic constructs or compositions comprising thereof can be administered to a mammal to alter gene expression or to re-engineer or alter the genome. For example, the genetic constructs or compositions comprising thereof can be administered to a mammal to correct the *dystrophin* gene in a mammal. The mammal can be human, non-human primate, cow, pig, sheep, goat, antelope, bison, water buffalo, bovids, deer, hedgehogs, elephants, llama, alpaca, mice, rats, or chicken, and preferably human, cow, pig, or chicken.

The vector encoding at least one Cas9 molecule and a pair of two gRNA molecules can be delivered to the mammal by DNA injection (also referred to as DNA vaccination) with and without *in vivo* electroporation, liposome mediated, nanoparticle facilitated, and/or recombinant vectors. The recombinant vector can be delivered by any viral mode. The viral mode can be recombinant lentivirus, recombinant adenovirus, and/or recombinant adeno-associated virus.

The disclosed vector or a composition comprising thereof can be introduced into a cell to genetically correct a *dystrophin* gene (e.g., human *dystrophin* gene). The disclosed vector or composition may be introduced into a myoblast cell from a DMD patient. The vector or composition comprising thereof may be introduced into a fibroblast cell from a DMD patient, and the genetically corrected fibroblast cell can be treated with MyoD to induce differentiation into myoblasts, which can be implanted into subjects, such as the damaged muscles of a subject to verify that the corrected dystrophin protein is functional and/or to treat the subject. The modified cells can also be stem cells, such as induced pluripotent stem cells, bone marrow-derived progenitors, skeletal muscle progenitors, human skeletal myoblasts from DMD patients, CD 133⁺ cells, mesoangioblasts, and MyoD- or Pax7- transduced cells, or other myogenic progenitor cells. For example, the CRISPR/Cas9-based system may cause neuronal or myogenic differentiation of an induced pluripotent stem cell.

### 6. Routes of Administration

The disclosed vector or a composition comprising thereof can be administered to a subject by different routes including orally, parenterally, sublingually, transdermally, rectally, transmucosally, topically, via inhalation, via buccal administration, intrapleurally, intravenously, via intraarterial administration, via intraperitoneal administration, subcutaneously, via intramuscular administration, via intranasal administration, via intrathecal administration, via intraarticular administration, and combinations thereof. The presently disclosed vector or a composition may be administered to a subject (e.g., a subject suffering from DMD) intramuscularly, intravenously or a combination thereof. For veterinary use, the presently disclosed vector or composition can be administered as a suitably acceptable formulation in accordance with normal veterinary practice. The veterinarian may readily determine the dosing regimen and route of administration that is most appropriate for a particular animal. A presently disclosed vector or a composition comprising thereof can be administered by traditional syringes, needleless injection devices, "microprojectile bombardment gone guns", or other physical methods such as electroporation ("EP"), "hydrodynamic method", or ultrasound.

The presently disclosed vector or a composition comprising thereof can be delivered to the mammal by several technologies including DNA injection (also referred to as DNA vaccination) with and without *in vivo* electroporation, liposome mediated, nanoparticle facilitated, recombinant vectors such as recombinant lentivirus, recombinant adenovirus, and recombinant adenovirus associated virus. the presently disclosed vector or a composition comprising thereof can be injected into the skeletal muscle or cardiac muscle. For example, the presently disclosed vector or a composition comprising thereof can be injected into the tibialis anterior muscle.

### 7. Cell types

Any of these delivery methods and/or routes of administration can be utilized with a myriad of cell types, for example, those cell types currently under invtestigation for cell-based therapies of DMD, including, but not limited to, immortalized myoblast cells, such as wild-type and DMD patient derived lines, for example Δ48-50 DMD, DMD 8036 (del48-50), C25C14 and DMD-7796 cell lines, primal DMD dermal fibroblasts, induced pluripotent stem cells, bone marrow-derived progenitors, skeletal muscle progenitors, human skeletal myoblasts from DMD patients, CD 133⁺ cells, mesoangioblasts, cardiomyocytes, hepatocytes, chondrocytes, mesenchymal progenitor cells, hematopoetic stem cells, smooth muscle cells, and MyoD- or Pax7-transduced cells, or other myogenic progenitor cells. Immortalization of human myogenic cells can be used for clonal derivation of genetically corrected myogenic cells. Cells can be modified *ex vivo* to isolate and expand clonal populations of immortalized DMD myoblasts that induce a genetically corrected *dystrophin* gene and are free of other nuclease-introduced mutations in protein coding regions of the genome.

### EXAMPLES

Having now described the present disclosure in detail, the same will be more clearly understood by reference to the following examples, which are merely intended only to illustrate some aspects and embodiments of the disclosure, and should not be viewed as limiting to the scope of the disclosure.

The presently disclosed subject matter has multiple aspects, illustrated by the following non-limiting examples.

### Example 1 - Deletion of exon 51 of human dystrophin genes by AAV vectors in immortalized DMD patient myoblasts

12 plasmid AAV vectors, each of which encodes an *S. aureus* Cas9 molecule and one pair of gRNA molecules selected from the 12 gRNA pairs list in Table 1, were made. The codon optimized nucleic acid sequence encoding the *S. aureus* Cas9 molecule Cas9 molecule is set forth in SEQ ID NO: 29. Among the 12 plasmid AAV vectors, three plasmid AAV vectors encoding gRNA pairs (84+68), (82+68), and (62+38), respectively, were transfected into HEK293T cells, and were electroporated into immortalized human DMD patient myoblasts. Cells were differentiated, and RNA and protein were collected. End point PCR and droplet digital PCR was performed on gDNA and cDNA, western blot on the protein.

### Methods and Materials

Immortalized human DMD patient myoblasts including a deletion of exons 48-50 were cultured in skeletal muscle media (PromoCell) supplemented with 20% FBS, 1% antibiotic, 1% GlutaMAX, 50 µg/mL fetuin, 10 ng/ul human epidermal growth factor, 1 ng/ml basic human fibroblast growth factor, and 10 µg/ml human insulin. The plasmids were electroporated into immortalized human DMD patient myoblasts, e.g., immortalized human DMD patient myoblasts were electroporated with 10 µg plasmid using the Gene Pulser XCell with PBS as an electroporation buffer using previously optimized conditions. Cells were incubated for three days post electroporation, and then genomic DNAwas harvested and collected using the DNEasy Blood and Tissue Kit. 50 ng of genomic DNA was used for droplet digital PCR ("ddPCR"). The deletion efficiencies of the plasmids were measured by ddPCR, as described in PCT Application No. PCT/US16/025738. 100 ng of gemomic DNA was used for end point PCR to detect deletion bands. Sequencing was performed for detected deletion bands.

The remaining electroporated myoblasts were differentiated into myofibers by replacing the standard culturing medium with DMEM supplmented with 1% antibiotic and 1% insulin-transferrin-selenium. Cells were differentiated for 6-7 days, then RNA was isolated using the RNEasy Plus Mini Kit. RNA was reversed transcribed to cDNA using the VILO cDNA synthesis kit. Protein was harvested from differentiated cells by collection and lysis in RIPA buffer with protease inhibitor cocktail. Samples were run on a 4-12% NuPAGE Bis-Tris gel in MES buffer. Proteins were transferred to a nitrocellulose membrane, then the Western blot was blocked for at least 1 hour. The primary antibody used for dystrophin expression was MANDYS8 at 1: 1000.

### Results

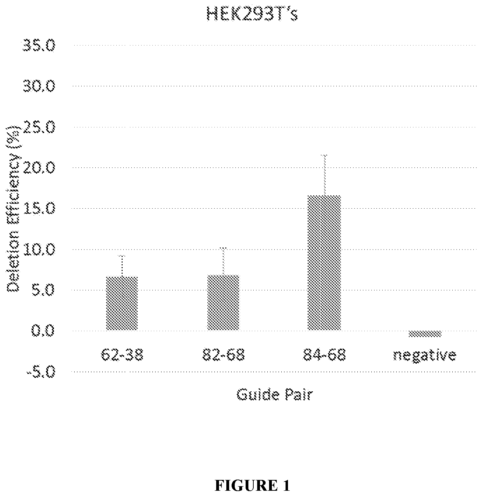

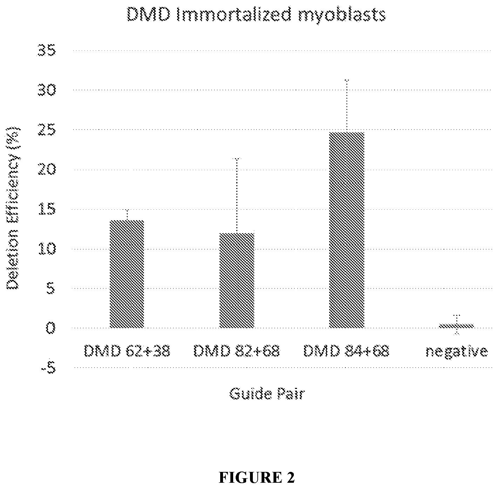

The deletion efficiencies of the three plasmic AAV vectors encoding gRNA pairs (84+68), (82+68), and (62+38), respectively, in transfected HEK293T cells are shown in Figure 1. The deletion efficiencies of these three plasmic AAV vectors in immortalized DMD patient myoblasts are shown in Figure 2. For both transfected H293T cells and for immortalized DMD patient myoblasts, *S. aureus* Cas9 was used as a negative control. The myoblast deletion effiency correlated well with HEK293T cells deletion efficiency.

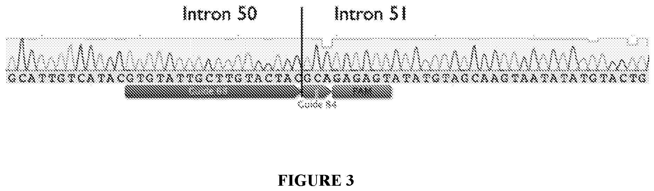

Deletion bands were detected for the plasmid AAV vectors. The sequencing result for the deletion band by the plasmid AAV vectors encoding the gRNA pair (84+68) is shown in Figure 3. As shown in Figure 3, the plasmid AAV vector encoding the gRNA pair (84+68) mediated precise expected deletion of exon 51 of human *dystrophin* gene.

### Example 2 - Deletion of exon 51 of human dystrophin genes by AAV vectors in humanized mice including human dystrophin gene

Mouse models, including humanized mouse models, are considered useful in evaluating and adapting compositions and methods, such as those disclosed herein, for the treatment or prevention of disease in human and animal subjects. *See*, *e.g.*, E. Nelson et al., Science 10.1126/science.aad5143 (2015),M. Tabebordbar et al., Science (2015). 10.1126/science.aad5177, and Long et al., Science (2016;Jan 22); 351(6271):400-403. For example, skilled artisans will appreciate that changes in genotype and/or phenotype observed in humanized mouse models of DMD can be predictive of changes in genotype and/or phenotype in human patients treated with the compositions and methods of the present disclosure. In particular, a method or composition that is efficacious in rescuing a disease (or disease-like) genotype or phenotype in a humanized mouse model can be readily adapted by those of skill in the art to therapeutic use in human subjects, and such adaptations are within the scope of the present disclosure.

One humanized mouse model of DMD is based on the *mdx* mouse model described by C. E. Nelson et al., Science 10.1126/science.aad5143 (2015). The *mdx* mouse carries a nonsense mutation in exon 23 of the mouse *dystrophin* gene, which results in production of a full-length dystrophin mRNA transcript and encodes a truncated dystrophin protein. These molecular changes are accompanied by functional changes including reduced twitch and tetanic force in *mdx* muscle. The *mdx* mouse has been humanized by the addition of a full-lenth human *dystrophin* transgene comprising a deletion of exon 52 ("*mdx* Δ52 mouse").

The *mdx* Δ52 mice were made by injecting a CRISPR/Cas9 system including a *S*. *pyogenes* Cas9 molecule and a pair of gRNAs targeting intron 51 and intron 52 of the human *dystrophin* gene, respectively, to the embryos of *mdx* mice containing the human *dystrophin* transgene. No dystrophin protein was detected in the heart and tibialis anterior muscle of the *mdx* Δ52 mice.

In one experiment, an AAV vector encoding an *s*. *aureus* Cas9 and a pair of gRNAs comprising targeting sequences set forth in Table 1 is administered to, e.g. the right tibilalis of each of a plurality of *mdx* Δ52 mice. The left tibialis anterior muscles of the *mdx* Δ52 mice are used as contralateral controls, receiving no treatment or an empty vector. At various timepoints following administration of the vector, mice are euthanized and tissues are harvested for histology, protein extraction and/or nucleic acid extraction. The degree of editing, and cellular and molecular changes following the treatment may be assessed as described above and in Nelson *et al.*

In another experiment, AAV vectors encoding Cas9 and gRNA pairs as described above are administered systematically to the *mdx* Δ52 mice, for instance by intravascular injection, and analyzed in more or less the same manner described above. The results of this experiment, the experiment described above, and/or other similar experiments may be used to evaluate and rank-order particular guide-pairs for therapeutic efficacy, to design and/or optimize AAV vectors and dosing protocols, and to assses the potential clinical utility of particular compositions or methods according to the present disclosure.

It is understood that the foregoing detailed description and accompanying examples are merely illustrative and are not to be taken as limitations upon the scope of the presently disclosed subject matter, which is defined solely by the appended claims and their equivalents. Various changes and modifications to the disclosed embodiments will be apparent to those skilled in the art.

## Claims

1. A vector encoding:
(a) a first guide RNA (gRNA) molecule,
(b) a second gRNA molecule, and
(c) at least one Cas9 molecule that recognizes a Protospacer Adjacent Motif (PAM) of either NNGRRT (SEQ ID NO: 24) or NNGRRV (SEQ ID NO: 25),
wherein the at least one Cas9 molecule is an *S. aureus* Cas9 molecule;
and wherein the first gRNA molecule comprises a targeting domain consisting of a nucleotide sequence as set forth in SEQ ID NO: 1, and the second gRNA molecule comprises a targeting domain consisting of a nucleotide sequence as set forth in SEQ ID NO: 2.

2. The vector of claim 1, wherein the vector is configured to form a first and a second double strand break in a first and a second intron flanking exon 51 of the human *DMD* gene, respectively, thereby deleting a segment of the dystrophin gene comprising exon 51.

3. The vector of claim 2, wherein the segment has a length of about 800-900, about 1500-2600, about 5200-5500, about 20,000-30,000, about 35,000-45,000, or about 60,000-72,000 base pairs.

4. The vector of any one of claims 1-3, wherein the at least one Cas9 molecule is a mutant *S. aureus* Cas9 molecule.

5. The vector of any one of claims 1-4, wherein, the vector is a viral vector.

6. The vector of claim 5, wherein the vector is an Adeno-associated virus (AAV) vector.

7. The vector of any one of claims 1-6 for use in a medicament.

8. A composition comprising the vector of any one of claims 1-6.

9. A cell comprising the vector of any one of claims 1-6.

10. A CRISPR/Cas9-based system comprising:
(a) a first guide RNA (gRNA) molecule,
(b) a second gRNA molecule, and
(c) at least one Cas9 molecule that recognizes a Protospacer Adjacent Motif (PAM) of either NNGRRT (SEQ ID NO: 24) or NNGRRV (SEQ ID NO: 25),
wherein the at least one Cas9 molecule is an *S. aureus* Cas9 molecule;
and wherein the first gRNA molecule comprises a targeting domain consisting of a nucleotide sequence as set forth in SEQ ID NO: 1, and the second gRNA molecule comprises a targeting domain consisting of a nucleotide sequence as set forth in SEQ ID NO: 2.

11. The CRISPR/Cas9-based system of claim 10, wherein the CRISPR/Cas9-based system is configured to form a first and a second double strand break in a first and a second intron flanking exon 51 of the human *DMD* gene, respectively, thereby deleting a segment of the dystrophin gene comprising exon 51.

12. The CRISPR/Cas9-based system of claim 11, wherein the segment has a length of 800-900, 1500-2600, 5200-5500, 20,000-30,000, 35,000-45,000, or 60,000-72,000 base pairs.

13. The CRISPR/Cas9-based system of any one of claims 10-12, wherein the at least one Cas9 molecule is a mutant *S. aureus* Cas9 molecule.

14. The CRISPR/Cas9-based system of any one of claims 10-13, for use in a medicament.

15. The vector of any one of claims 1-7, or the CRISPR/Cas9-based system of any one of claims 10 -14, for use in the treatment of Duchenne Muscular Dystrophy.

## Patentansprüche

1. Vektor, codierend:
(a) ein erstes Guide-RNA-Molekül (gRNA-Molekül);
(b) ein zweites gRNA-Molekül, und
(c) mindestens ein Cas9-Molekül, das ein Protospacer Adjacent Motif (PAM) von entweder NNGRRT (SEQ ID Nr. 24) oder NNGRRV (SEQ ID Nr. 25) erkennt,
wobei das mindestens eine Cas9-Molekül ein *S.-aureus*-Cas9-Molekül ist;
und wobei das erste gRNA-Molekül eine Targeting-Domäne umfasst, die aus einer wie in SEQ ID Nr. 1 dargelegten Nukleotidsequenz besteht, und das zweite gRNA-Molekül eine Targeting-Domäne umfasst, die aus einer wie in SEQ ID Nr. 2 dargelegten Nukleotidsequenz besteht.

2. Vektor nach Anspruch 1, wobei der Vektor dazu konfiguriert ist, einen ersten und einen zweiten Doppelstrangbruch in einem ersten bzw. einem zweiten Intron flankierenden Exon 51 des humanen DMD-Gens zu bilden, wodurch ein Segment des Dystrophin-Gens, das das Exon 51 umfasst, deletiert wird.

3. Vektor nach Anspruch 2, wobei das Segment eine Länge von etwa 800-900, etwa 1500-2600, etwa 5200-5500, etwa 20.000-30.000, etwa 35.000-45.000 oder etwa 60.000-72.000 Basenpaaren aufweist.

4. Vektor nach einem der Ansprüche 1 bis 3, wobei das mindestens eine Cas9-Molekül ein mutiertes *S.-aureus*-Cas9-Molekül ist.

5. Vektor nach einem der Ansprüche 1 bis 4, wobei der Vektor ein viraler Vektor ist.

6. Vektor nach Anspruch 5, wobei der Vektor ein Adenoassoziierter Virus-Vektor (AAV-Vektor) ist.

7. Vektor nach einem der Ansprüche 1 bis 6 zur Verwendung in einem Arzneimittel.

8. Zusammensetzung, umfassend den Vektor nach einem der Ansprüche 1 bis 6.

9. Zelle, umfassend den Vektor nach einem der Ansprüche 1 bis 6.

10. System auf CRISPR/Cas9-Basis, umfassend:
(a) ein erstes Guide-RNA-Molekül (gRNA-Molekül);
(b) ein zweites gRNA-Molekül, und
(c) mindestens ein Cas9-Molekül, das ein Protospacer Adjacent Motif (PAM) von entweder NNGRRT (SEQ ID Nr. 24) oder NNGRRV (SEQ ID Nr. 25) erkennt,
wobei das mindestens eine Cas9-Molekül ein *S.-aureus*-Cas9-Molekül ist;
und wobei das erste gRNA-Molekül eine Targeting-Domäne umfasst, die aus einer wie in SEQ ID Nr. 1 dargelegten Nukleotidsequenz besteht, und das zweite gRNA-Molekül eine Targeting-Domäne umfasst, die aus einer wie in SEQ ID Nr. 2 dargelegten Nukleotidsequenz besteht.

11. System auf CRISPR/Cas9-Basis nach Anspruch 10, wobei das System auf CRISPR/Cas9-Basis dazu konfiguriert ist, einen ersten und einen zweiten Doppelstrangbruch in einem ersten bzw. einem zweiten Intron flankierenden Exon 51 des humanen DMD-Gens zu bilden, wodurch ein Segment des Dystrophin-Gens, das das Exon 51 umfasst, deletiert wird.

12. System auf CRISPR/Cas9-Basis nach Anspruch 11, wobei das Segment eine Länge von etwa 800-900, 1500-2600, 5200-5500, 20.000-30.000, 35.000-45.000 oder 60.000-72.000 Basenpaaren aufweist.

13. System auf CRISPR/Cas9-Basis nach einem der Ansprüche 10 bis 12, wobei das mindestens eine Cas9-Molekül ein mutiertes *S.-aureus*-Cas9-Molekül ist.

14. System auf CRISPR/Cas9-Basis nach einem der Ansprüche 10 bis 13 zur Verwendung in einem Arzneimittel.

15. Vektor nach einem der Ansprüche 1 bis 7 oder System auf CRISPR/Cas9-Basis nach einem der Ansprüche 10 bis 14 zur Verwendung in der Behandlung von Duchenne-Muskeldystrophie.

## Revendications

1. Vecteur codant :
(a) une première molécule d'ARN de guidage (ARNg),
(b) une deuxième molécule d'ARNg,
(c) au moins une molécule de Cas9 qui reconnaît un motif adjacent proto-espaceur (PAM) de soit NNGRRT (SEQ ID NO : 24) soit NNGRRV (SEQ ID NO : 25),
dans lequel l'au moins une molécule de Cas9 est une molécule de Cas9 de *S. aureus* ;
et dans lequel la première molécule d'ARNg comprend un domaine de ciblage consistant en une séquence de nucléotides telle qu'indiquée dans la SEQ ID NO : 1, et la deuxième molécule d'ARNg comprend un domaine de ciblage consistant en une séquence de nucléotides telle qu'indiquée dans la SEQ ID NO : 2.

2. Vecteur selon la revendication 1, lequel vecteur est configuré pour former des première et deuxième ruptures de double brin dans des premier et deuxième introns flanquant l'exon 51 du gène *DMD* humain, respectivement, en supprimant ainsi un segment du gène de la dystrophine comprenant l'exon 51.

3. Vecteur selon la revendication 2, dans lequel le segment a une longueur d'environ 800 à 900, d'environ 1 500 à 2 600, d'environ 5 200 à 5 500, d'environ 20 000 à 30 000, d'environ 35 000 à 45 000, ou d'environ 60 000 à 72 000 paires de bases.

4. Vecteur selon l'une quelconque des revendications 1 à 3, dans lequel l'au moins une molécule de Cas9 est une molécule de Cas9 de *S. aureus* mutante.

5. Vecteur selon l'une quelconque des revendications 1 à 4, lequel vecteur est un vecteur viral.

6. Vecteur selon la revendication 5, lequel vecteur est un vecteur de type virus adéno-associé (AAV).

7. Vecteur selon l'une quelconque des revendications 1 à 6, pour une utilisation en tant que médicament.

8. Composition comprenant le vecteur de l'une quelconque des revendications 1 à 6.

9. Cellule comprenant le vecteur de l'une quelconque des revendications 1 à 6.

10. Système basé sur CRISPR/Cas9 comprenant :
(a) une première molécule d'ARN de guidage (ARNg),
(b) une deuxième molécule d'ARNg,
(c) au moins une molécule de Cas9 qui reconnaît un motif adjacent proto-espaceur (PAM) de soit NNGRRT (SEQ ID NO : 24) soit NNGRRV (SEQ ID NO : 25),
dans lequel l'au moins une molécule de Cas9 est une molécule de Cas9 de *S. aureus* ;
et dans lequel la première molécule d'ARNg comprend un domaine de ciblage consistant en une séquence de nucléotides telle qu'indiquée dans la SEQ ID NO : 1, et la deuxième molécule d'ARNg comprend un domaine de ciblage consistant en une séquence de nucléotides telle qu'indiquée dans la SEQ ID NO : 2.

11. Système basé sur CRISPR/Cas9 selon la revendication 10, lequel système basé sur CRISPR/Cas9 est configuré pour former des première et deuxième ruptures de double brin dans des premier et deuxième introns flanquant l'exon 51 du gène *DMD* humain, respectivement, en supprimant ainsi un segment du gène de la dystrophine comprenant l'exon 51.

12. Système basé sur CRISPR/Cas9 selon la revendication 11, dans lequel le segment a une longueur d'environ 800 à 900, 1 500 à 2 600, 5 200 à 5 500, 20 000 à 30 000, 35 000 à 45 000, ou 60 000 à 72 000 paires de bases.

13. Système basé sur CRISPR/Cas9 selon l'une quelconque des revendications 10 à 12, dans lequel l'au moins une molécule de Cas9 est une molécule de Cas9 de *S. aureus* mutante.

14. Système basé sur CRISPR/Cas9 selon l'une quelconque des revendications 10 à 13, pour une utilisation en tant que médicament.

15. Vecteur selon l'une quelconque des revendications 1 à 7, ou système basé sur CRISPR/Cas9 selon l'une quelconque des revendications 10 à 14, pour une utilisation dans le traitement de la dystrophie musculaire de Duchenne.
